(19) Europäisches Patentamt
European Patent Office
Office européen des brevets

(11) EP 0 985 442 A2

(12) **EUROPÄISCHE PATENTANMELDUNG**

(43) Veröffentlichungstag:
**15.03.2000 Patentblatt 2000/11**

(21) Anmeldenummer: **99113689.6**

(22) Anmeldetag: **15.07.1999**

(51) Int Cl.⁷: **B01D 71/70**, C08G 77/00, C08L 83/00, C08F 230/08, A61M 1/16

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **10.09.1998 DE 19841439**

(71) Anmelder: **FRAUNHOFER-GESELLSCHAFT ZUR FÖRDERUNG DER ANGEWANDTEN FORSCHUNG E.V.
80636 München (DE)**

(72) Erfinder:
- **Wolter, Herbert, Dr.
97950 Gerchsheim (DE)**
- **Ballweg, Thomas, Dipl.-Phys.
97877 Wertheim (DE)**
- **Storch, Werner, Dipl.-Phys.
97204 Höchberg (DE)**

(54) **Oxygenatormembran**

(57) Die Erfindung betrifft eine Oxygenatormembran auf der Basis von organisch modifizierten Kieselsäurepolykondensafen und ein Verfahren zu deren Herstellung. Die Oxygenatormembran ist dadurch erhältlich, dass man eine viskose bis harzartige Flüssigkeit nach üblichen Methoden zu einer Membranen verarbeitet, diese gegebenenfalls trocknet und thermisch und/oder strahlungsinduziert und/oder chemisch induziert härtet. Die viskose bis harzartige Flüssigkeit wird erhalten

a) durch hydrolytische Polykondensation von einer oder mehreren Verbindungen der allgemeinen Formel I und/ oder II und/oder III und/oder IV und/oder von diesen durch hydrolytische Kondensation abgeleiteten Vorkondensaten und gegebenenfalls von einer oder mehreren Verbindungen der allgemeinen Formel V, und gegebenenfalls b) durch Zugabe von einem oder mehreren copolymerisierbaren und/oder (poly) addierbaren Monomeren und/ oder Oligomeren und/oder von einem oder mehreren Härtungskatalysatoren.

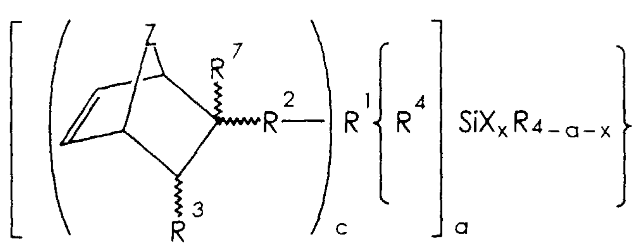

(I),

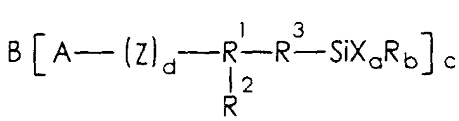

(II),

$\{X_aR_bSi[(R'A)_c]_{(4-a-b)}\}_xB$ (III)  $Y_aSiX_xR_{4-a-x}$ (IV)  $X_aSiR_{4-a}$ (V)

EP 0 985 442 A2

## Beschreibung

Die Erfindung betrifft eine Oxygenatormembran auf der Basis von organisch modifizierten Kieselsäurepolykondensaten und ein Verfahren zu deren Herstellung.

Oxygenatoren für Humanblut werden z.B. während Herzoperationen oder bei der Behandlung reversibler pulmonaler Insuffizienzen eingesetzt und sollen temporär die natürliche Funktion der Lunge übernehmen. Blut muss in ausreichendem Maße mit Sauerstoff versorgt werden, gleichzeitig muss das durch Stoffwechselvorgänge im Körper entstandene Kohlendioxid entfernt werden. Das einfachste Verfahren zur Sauerstoff-Anreicherung im Blut wird mit sog. "Bubble"-Oxygenatoren (BO) durchgeführt, die bezüglich ihres Aufbaus einfach und kostengünstig sind. Beim BO wird in das durch eine Säule oder speziell geformte Kunststoffbeutel strömende Blut Sauerstoff eingeblasen. Der Gasaustausch findet an der Grenzfläche zwischen Blut und Gasblasen statt. Der Gasaustausch auf diese Art und Weise hat den großen Nachteil, dass durch den direkten Kontakt Gas-Blut Thrombenbildung induziert wird. Die sich bildenden Thromben stellen eine Emboliegefahr dar und beeinflussen die Blutgerinnung in negativer Weise. Zum anderen ergibt sich durch die Thrombenbildung an der Grenzfläche der Gasblasen eine drastische Verminderung der wirksamen Austauschfläche. Außerdem kann nicht verhindert werden, dass Mikrogasblasen in den Blutkreislauf des Körpers gelangen. Aus diesen Gründen ist die Patientenbelastung relativ hoch, so dass die Einsatzzeit derartiger Oxygenatoren auf 1 bis 1.5 Stunden begrenzt ist.

Es werden vornehmlich Membran-Oxygenatoren (MO) eingesetzt, da mit diesen die natürliche Funktion der Lunge besser nachgebildet werden kann.

Bei der extrakorporalen Membran-Oxygenierung (EMCO) werden üblicherweise hydrophobe Membranen eingesetzt. Ein hydrophober Oxygenator ist z.B. in der DE 3129064 A1 beschrieben. Dort wird der Einsatz von hydrophoben Membranmaterialien, beispielsweise in Form von Hohlfasern, zur Oxygenierung vorgeschlagen. Dabei wird auf der einen Seite einer hydrophoben Membran Blut im extrakorporalen Kreislauf vorbeigeführt, während an der anderen Seite der Membran im Gegenstrom Sauerstoff zugeführt wird, so daß über die Poren der Membran hinweg ein $CO_2$/$O_2$-Austausch erfolgen kann.

Es werden üblicherweise zwei Typen von hydrophoben Membranen eingesetzt, nämlich Membranen, die aus einem per se hydrophoben Material , beispielsweise Polypropylen, bestehen und Membranen, deren Oberfläche mit einem Hydrophobierungsmittel, beispielsweise einem Silicon, hydrophob gemacht wurden.

Hydrophobe Membranen aus hydrophoben Materialien weisen vergleichsweise große Poren von mehreren 100 bis 1000 nm auf und liegen meist in Form von mehreren 1000 Hohlfäden vor, was in einer aktiven Oberfläche bis zu 6 $m^2$ resultiert. Das Blut fließt dabei entweder in den Hohlfäden oder aber auf der Außenseife des Hohlfadens, während das auszutauschende Gas auf der gegenüberliegenden Seite im Gegenstrom vorbeigeführt wird. Diese Membranen werden üblicherweise in Herz-Lungen-Maschinen eingesetzt.

Hydrophobierte Membranen bestehen aus einer dünnen Schicht Silicon auf einer porösen Trägerstruktur und werden für die Langzeit-EMCO-Behandlung eingesetzt. Zwar sind hydrophobe Membranen im Vergleich zu siliconisierten Membranen effektiver, da die Diffusion durch Poren wesentlich schneller abläuft. Jedoch haben Membranen aus hydrophobem porösen Material einen signifikanten Nachteil in der Langzeittherapie. Dieser Nachteil besteht im Lecken der Membran, da sich die Poren trotz ihrer hydrophoben Struktur mit wässrigen Plasmabestandteilen füllen, was zu einer Hydrophilierung bzw. Benetzung der Membranoberfläche führt.

Nach dem Stand der Technik wird als Membranmaterial in Hohlfaser-Oxygenatoren im allgemeinen mikroporöses Polypropylen, z.T. auch Polyethylen, eingesetzt. Mit einer definierten durchgängigen Porosität versehene Polypropylen-Hohlfasern sind direkt nur durch sehr aufwendige Spinnprozesse (z.B. Lösungsnassspinnen) oder durch nachträgliche und somit zusätzliche Verfahrensschritte zugänglich. Werden entsprechend modifizierte Hohlfasern zum Gasaustausch in fluiden Systemen verwendet, besteht die Gefahr des Durchtritts der fluiden Phase. Beim $O_2$/$CO_2$-Austausch im Blut mittels Oxygenatoren bergen deshalb die Poren ein erhebliches Gefahrenpotential. Insbesondere bei längeren Einsatzzeiten wird des öfteren ein Durchtritt von Blut durch die Poren beobachtet. Ebenso können Gasbläschen zur Gegenseite übertreten und dadurch Mikrotromben bilden.

Sehr hohe Gaspermeationswerte ohne Porosität sind nur mit ganz speziellen Polymeren, z.B. mit Siliconen oder substituierten Polysilylpropinen, realisierbar. Die hohe Gasdurchlässigkeit wird allerdings durch extreme Einbußen bei den mechanischen Eigenschaften erkauft. Mit steigender Permeabilität nimmt die Festigkeit und der E-Modul ab, d.h. das Material wird zunehmend weicher. Mechanisch ausreichend stabile Hohlfasern mit sehr geringer Wandstärke und hoher Gasdurchlässigkeit sind somit nicht mehr möglich. Hohlfasern mit einer über weite Bereiche einstellbaren Permeabilität sind nur auf der Basis sehr unterschiedlicher Polymertypen in Verbindung mit verschiedenen Produktionsverfahren möglich.

Aufgabe der vorliegenden Erfindung ist es, Membranen für Oxygenatoren bereit zu stellen. Die Permeabilität und Flexibilität der Membranen soll über weite Bereiche variiert und den Anforderungen des jeweiligen Anwendungsfalles angepasst sein. Ferner sollen die Membranen bei hoher mechanischer Stabilität hohe Gas-Permeabilitäten zeigen, ohne dass die Gefahr des Durchtritts der fluiden Phase besteht. Auch bei hohen Gas-Permeationswerten sollen

die Membranen noch freitragend sein. Ferner sollen die Membranen toxikologisch unbedenklich sein.

Aufgabe der vorliegenden Erfindung ist es auch, ein Verfahren bereit zustellen, mit dem Membranen für Oxygenatoren gefertigt werden können, deren Eigenschaftsprofil in weiten Bereichen variierbar ist. Durch einfache Variation der Verfahrensschritte sollen die chemischen und physikalischen Eigenschaften der Membran in weiten Bereichen den Anforderungen des jeweiligen Anwendungsfalles angepasst werden können. Das Verfahren soll einfach, schnell und kostengünstig durchführbar sein. Mit dem Verfahren sollen Membranen gefertigt werden können, die den oben genannten Anforderungen entsprechen. Ferner soll das Verfahren auch für die Endlosproduktion von Hohlfaser- und Flachmembranen geeignet sein. Außerdem sollen die für verschiedene Anwendungen oftmals erforderlichen Oberflächen-Modifikationen, z.B. zur Vermeidung von Blutkoagulationen, zur Einstellung der Polarität, des Adsorptionsverhaltens etc., sowohl während der Materialsynthese, d.h. in situ, als auch nachträglich realisiert werden können.

Gelöst wird diese Aufgabe durch Oxygenatormembranen, die dadurch erhältlich sind, dass man eine mehr oder weniger viskose Flüssigkeit oder ein Harz nach üblichen Methoden zu Membranen verarbeitet und diese gegebenenfalls trocknet. Die resultierenden Membranen werden dann thermisch und/oder strahlungsinduziert und/oder chemisch induziert gehärtet.

Die gering viskose bis harzartige Flüssigkeit, aus der die Membranen gefertigt werden, wird erhalten

a) durch hydrolytische Polykondensation von

- einer oder mehreren Verbindungen der allgemeinen Formel I, und/oder
- einer oder mehreren Verbindungen der allgemeinen Formel II, und/oder
- einer oder mehreren Verbindungen der allgemeinen Formel III, und/oder
- einer oder mehreren Verbindungen der allgemeinen Formel IV, und/oder
- von den Verbindungen der Formeln I bis IV abgeleiteten Vorkondensaten und gegebenenfalls
- einer oder mehreren Verbindungen der allgemeinen Formel V, und/oder von diesen abgeleiteten Vorkondensaten,

und gegebenenfalls

b) durch Zugabe von

- einem oder mehreren copolymerisierbaren und/oder (poly)addierbaren Monomeren und/oder Oligomeren,
- und/oder von einem oder mehreren Härtungskatalysatoren.

Die hydrolytische Polykondensation wird durch Zugabe von Wasser oder von Feuchtigkeit und gegebenenfalls in Anwesenheit eines Lösungsmittels und/oder eines Kondensationskatalysators durchgeführt. Bezogen auf die Monomere liegt das molare Verhältnis der Summe der Verbindungen der Formeln I, II, III und IV zu Verbindungen der Formel V zwischen 1:0 und 1: 20.

Die zur Herstellung der erfindungsgemäßen Membranen eingesetzte Flüssigkeit bzw. das eingesetzte Harz stellen also ein Polykondensat aus hydrolytisch kondensierten Siliciumverbindungen der Formeln I und/oder II und/oder III und/oder IV und gegebenenfalls V dar, wobei das Polykondensat gegebenenfalls noch Wasser und/oder Lösungsmittel und/oder die oben genannten Zusatzstoffe erhält. In Abhängigkeit von der Viskosität des Polykondensates kann man von einer mehr oder weniger viskosen Flüssigkeit oder von einem Harz reden.

In der allgemeinen Formel I

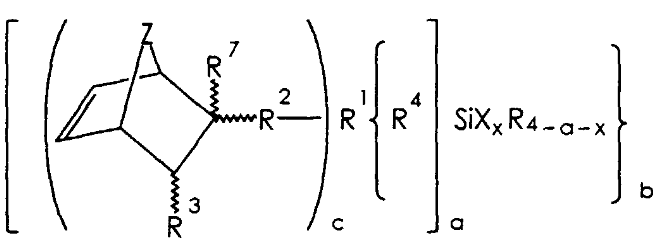

(I)

haben die Reste und Indices folgende Bedeutung, wobei bei Indices ≥ 2 die diesbezüglichen Reste gleich oder verschieden sind.

R = Alkyl, Alkenyl, Aryl, Alkylaryl oder Arylalkyl mit jeweils 1 bis 15 Kohlenstoff-Atomen, wobei diese Reste Sauerstoff- und/oder Schwefel-Atome und/ oder Ester- und/oder Carbonyl- und/oder Carboxyl- und/oder Amid- und/ oder Amino-Gruppen enthalten können.

$R^1$ = Alkylen, Arylen, Arylenalkylen oder Arylenalkylen mit jeweils 0 bis 15 Kohlenstoff-Atomen, wobei diese Reste Sauerstoff- und/oder Schwefel-Atome und/oder Ester- und/oder Carbonyl- und/oder Carboxyl- und/oder Amid- und/oder Amino-Gruppen enthalten können.

$R^2$ = Alkylen, Arylen, Arylenalkylen oder Arylenalkylen mit jeweils 0 bis 15 Kohlenstoff-Atomen, wobei diese Reste Sauerstoff- und/oder Schwefel-Atome und/oder Ester- und/oder Carbonyl- und/oder Carboxyl- und/oder Amid- und/oder Amino-Gruppen enthalten können.

$R^3$ = Wasserstoff, $R^2$-$R^1$-$R^4$-$SiX_xR_{3-x}$, Carboxyl-, Alkyl, Alkenyl, Aryl, Alkylaryl oder Arylalkyl mit jeweils 1 bis 15 Kohlenstoff-Atomen, wobei diese Reste Sauerstoff- und/oder Schwefel-Atome und/oder Ester- und/oder Carbonyl- und /oder Carboxyl- und/oder Amid- und/oder Amino-Gruppen enthalten können.

$R^4$ = $-(CHR^6-CHR^6)_n-$, mit n = 0 oder 1, $-CHR^6-CHR^6-S-R^5-$, $-CO-S-R^5-$, $-CHR^6-CHR^6-NR^6-R^5-$, $-Y-CS-NH-R^5-$, $-S-R^5$,$-Y-CO-NH-R^5-$, $-CO-O-R^5-$, $-Y-CO-C_2H_3(COOH)-R^5-$, $-Y-CO-C_2H_3(OH)-R^5-$ oder $-CO-NR^6-R^5-$.

$R^5$ = Alkylen, Arylen, Arylenalkylen oder Arylenalkylen mit jeweils 1 bis 15 Kohlenstoff-Atomen, wobei diese Reste Sauerstoff- und/oder Schwefel-Atome und/oder Ester- und/oder Carbonyl- und/oder Carboxyl- und/oder Amid- und/oder Amino-Gruppen enthalten können.

$R^6$ = Wasserstoff, Alkyl oder Aryl mit 1 bis 10 Kohlenstoff-Atomen.

$R^7$ = Wasserstoff, Alkyl, Alkenyl, Aryl, Alkylaryl oder Arylalkyl mit jeweils 1 bis 15 Kohlenstoff-Atomen, wobei diese Reste Sauerstoff- und/oder Schwefel-Atome und/oder Ester- und/oder Carbonyl- und/oder Carboxyl- und/ oder Amid- und/oder Amino-Gruppen enthalten können.

X = Wasserstoff, Halogen, Hydroxy, Alkoxy, Acyloxy, Alkylcarbonyl, Alkoxycarbonyl oder $NR''_2$, mit R'' = Wasserstoff, Alkyl, Alkylaryl oder Aryl.

Y = -O-, -S- oder $-NR^6-$.

Z = -O- oder $-(CHR^6)_m-$, mit m = 1 oder 2.

a = 1, 2 oder 3, mit b = 1 für a = 2 oder 3.

b = 1, 2 oder 3, mit a = 1 für b = 2 oder 3.

c = 1 bis 6.

x = 1, 2 oder 3, mit a+x = 2, 3 oder 4.

Organisch modifizierte Silane der allgemeinen Formel I, deren Herstellung sowie konkrete Beispiele sind in der DE 19627198 C2 ausführlichst beschrieben. Auf die Offenbarung der DE 19627198 C2 wird hier ausdrücklich Bezug genommen. In bevorzugten Ausführungsformen der erfindungsgemäßen Membranen werden organisch modifizierte Silane der allgemeinen Formel I und/oder davon abgeleitete Vorkondensate eingesetzt, in denen die Indices a und/oder b und/oder c den Wert 1 annehmen.

In der allgemeinen Formel II

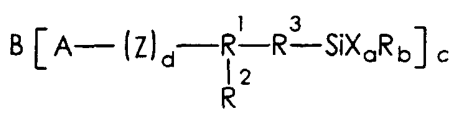

(II)

haben die Reste und Indices folgende Bedeutung, wobei bei Indices ≥ 2 die diesbezüglichen Reste gleich oder verschieden sind.

B = ein geradkettiger oder verzweigter organischer Rest mit mindestens einer C=C-Doppelbindung und 4 bis 50 Kohlenstoff-Atomen.

R = Alkyl, Alkenyl, Aryl, Alkylaryl oder Arylalkyl mit jeweils 1 bis 15 Kohlenstoff-Atomen, wobei diese Reste Sauerstoff- und/oder Schwefel-Atome und/oder Ester- und/oder Carbonyl- und/oder Carboxyl- und/oder Amid- und/ oder Amino-Gruppen enthalten können.

$R^3$ = Alkylen, Arylen, Arylenalkylen oder Alkylenarylen mit jeweils 0 bis 10 Kohlenstoff-Atomen, wobei diese Reste durch Sauerstoff- und/oder durch Schwefel-Atome und/oder durch Amino-Gruppen unterbrochen sein können.

X = Wasserstoff, Halogen, Hydroxy, Alkoxy, Acyloxy, Alkylcarbonyl, Alkoxycarbonyl oder $NR''_2$, mit R''= Wasserstoff, Alkyl, Aryl oder Alkylaryl.

A = O, S oder NH für d = 1 und Z = CO und

$R^1$ = Alkylen, Arylen oder Alkylenarylen mit jeweils 1 bis 10 Kohlenstoff-Atomen, wobei diese Reste durch Sauerstoff- und/oder durch Schwefel-Atome und/oder durch Amino-Gruppen unterbrochen sein können, und

$R^2$ = COOH oder H.

oder

A = O, S, NH oder COO für d = 1 und Z = CHR', mit R' = H, Alkyl, Aryl oder Alkylaryl, und

$R^1$ = Alkylen, Arylen oder Alkylenarylen mit jeweils 1 bis 10 Kohlenstoff-Atomen, wobei diese Reste durch Sauerstoff- und/oder durch Schwefel-Atome und/oder durch Amino-Gruppen unterbrochen sein können, und

$R^2$ = OH.

oder

A = O, S, NH oder COO für d = 0 und

$R^1$ = Alkylen, Arylen oder Alkylenarylen mit jeweils 1 bis 10 Kohlenstoff-Atomen, wobei diese Reste durch Sauerstoff- und/oder durch Schwefel-Atome und/oder durch Amino-Gruppen unterbrochen sein können, und

$R^2$ = OH.

oder

A = S für d = 1 und Z = CO und

$R^1$ = N und

$R^2$ = H.

a = 1,20 oder 3.

b = 0, 1 oder 2, mit a+b = 3.

c = 1, 2, 3 oder 4.

Organisch modifizierte Silane der allgemeinen Formel II, deren Herstellung sowie konkrete Beispiele sind in der DE 4416857 C1 ausführlichst beschrieben. Auf die Offenbarung der DE 4416857 C1 wird hier ausdrücklich Bezug genommen. In bevorzugten Ausführungsformen der erfindungsgemäßen Membranen werden organisch modifizierte Silane der allgemeinen Formel II und/oder davon abgeleitete Vorkondensate eingesetzt, in denen die Alkyl- und/oder Alkylen- und/oder Alkoxy-Gruppen 1 bis 4 Kohlenstoff-Atome aufweisen. In weiteren bevorzugten Ausführungsformen weist der Rest B der allgemeinen Formel II eine oder mehrere Acrylat- und/oder Methacrylat-Gruppen auf.

In der allgemeinen Formel III

$$\{X_aR_bSi[(R'A)_c]_{(4-a-b)}\}_xB \qquad (III)$$

haben die Reste und Indices folgende Bedeutung, wobei bei Indices ≥ 2 die diesbezüglichen Reste gleich oder verschieden sind.

A = O, S, PR", POR", NHC(O)O oder NHC(O)NR".

B = geradkettiger oder verzweigter organischer Rest, der sich von einer Verbindung B' mit mindestens einer (für c = 1 und A = NHC(O)O oder NHC(O)NR") bzw. mindestens zwei C=C-Doppelbindungen und 5 bis 50 Kohlenstoff-Atomen ableitet.

R = Alkyl, Alkenyl, Aryl, Alkylaryl oder Arylalkyl mit jeweils 1 bis 15 Kohlenstoff-Atomen, wobei diese Reste Sauerstoff- und/oder Schwefel-Atome und/oder Ester- und/oder Carbonyl- und/oder Carboxyl- und/oder Amid- und/oder Amino-Gruppen enthalten können.

R' = Alkylen, Arylen oder Alkylenarylen.

R" = Wasserstoff, Alkyl, Aryl oder Alkylaryl.

X = Wasserstoff, Halogen, Hydroxy, Alkoxy, Acyloxy, Alkylcarbonyl, Alkoxycarbonyl oder $NR''_2$.

a = 1, 2 oder 3.

b = 0, 1 oder 2.

c = 0 oder 1.

x = eine ganze Zahl, deren Maximalwert der Anzahl von Doppelbindungen in der Verbindung B' minus 1 entspricht, bzw. gleich der Anzahl von Doppelbindungen in der Verbindung B' ist, wenn c = 1 und A für NHC(O)O oder NHC(O)NR" steht.

Die obigen Alkyl- bzw. Alkenyl-Reste sind gegebenenfalls substituierte geradkettige, verzweigte oder cyclische Reste mit 1 bzw. 2 bis 20 Kohlenstoff-Atomen. Aryl steht für gegebenenfalls substituiertes Phenyl, Naphthyl oder Biphenyl, und die obigen Alkoxy-, Acyloxy-, Alkylcarbonyl-, Alkoxycarbonyl-, Alkylaryl-, Arylalkyl-, Arylen-, Alkylen- und Alkylenaryl-Reste leiten sich von den oben definierten Alkyl- und Aryl-Resten ab.

Organisch modifizierte Silane der allgemeinen Formel III, deren Herstellung sowie konkrete Beispiele sind in der DE 4011044 C2 ausführlichst beschrieben. Auf die Offenbarung der DE 4011044 C2 wird hier ausdrücklich Bezug genommen. In bevorzugten Ausführungsformen der erfindungsgemäßen Membranen werden Silane der allgemeinen Formel III und/oder davon abgeleitete Vorkondensate eingesetzt, in denen der Rest B eine oder mehrere Acrylat- und/oder Methacrylat-Gruppen aufweist.

In der allgemeinen Formel IV

$$Y_aSiX_xR_{4-a-x} \qquad (IV)$$

haben die Reste und Indices folgende Bedeutung, wobei bei Indices ≥ 2 die diesbezüglichen Reste gleich oder verschieden sind.

R = Alkyl, Alkenyl, Aryl, Alkylaryl oder Arylalkyl mit jeweils 1 bis 15 Kohlenstoff-Atomen, wobei diese Reste Sauerstoff- und/oder Schwefel-Atome und/oder Ester- und/oder Carbonyl- und/oder Carboxyl- und/oder Amid- und/oder Amino-Gruppen enthalten können.

X = Wasserstoff, Halogen, Hydroxy, Alkoxy, Acyloxy, Alkylcarbonyl, Alkoxycarbonyl oder $NR''_2$, mit R" = Wasserstoff, Alkyl, Aryl oder Alkylaryl.

Y = organischer Rest mit 1 bis 30, bevorzugt mit 1 bis 20 Kohlenstoff-Atomen und mit 1 bis 5, bevorzugt mit 1 bis 4 Mercapto-Gruppen.

a = 1, 2 oder 3.

x = 1, 2 oder 3, mit a+x = 2, 3 oder 4.

Die Alkyl-Reste sind z.B. geradkettige, verzweigte oder cyclische Reste mit 1 bis 20, insbesondere mit 1 bis 10 Kohlenstoff-Atomen und vorzugsweise niedere Alkyl-Reste mit 1 bis 6, besonders bevorzugt mit 1 bis 4 Kohlenstoff-Atomen. Spezielle Beispiele sind Methyl, Ethyl, n-Propyl, i-Propyl, n-Butyl, i-Butyl, s-Butyl, t-Butyl, n-Pentyl, n-Hexyl, Cyclohexyl, 2-Ethylhexyl, Dodecyl und Octadecyl. Die Alkenyl-Reste sind z.B. geradkettige, verzweigte oder cyclische Reste mit 2 bis 20, bevorzugt mit 2 bis 10 Kohlenstoff-Atomen und vorzugsweise niedere Alkenyl-Reste mit 2 bis 6 Kohlenstoff-Atomen, wie z.B. Vinyl, Allyl und 2-Butenyl. Bevorzugte Aryl-Reste sind Phenyl, Biphenyl und Naphthyl.

Die Alkoxy-, Acyloxy-, Alkylamino-, Dialkylamino-, Alkylcarbonyl-, Alkoxycarbonyl-, Arylaklyl-, Alkylaryl-, Alkylen- und Alkylenarylen-Reste leiten sich vorzugsweise von den oben genannten Alkyl- und Aryl-Resten ab. Spezielle Beispiele sind Methoxy, Ethoxy, n- und i-Propoxy, n-, i-, s- und t- Butoxy, Monomethylamino, Monoethyla-mino, Dimethylamino, Diethylamino, N-Ethylanilino, Acetyloxy, Propionyloxy, Methylcarbonyl, Ethylcarbonyl, Methoxycarbonyl, Ethoxycarbonyl, Benzyl, 2-Phenylethyl und Tolyl.

Die genannten Reste können gegebenenfalls einen oder mehrere Substituenten tragen, z.B. Halogen, Alkyl, Hydroxyalkyl, Alkoxy, Aryl, Aryloxy, Alkylcarbonyl, Alkoxycarbonyl, Furfuryl, Tetrahydrofurfuryl, Amino, Monoalkylamino, Dialkylamino, Trialkylammonium, Amido, Hydroxy, Formyl, Carboxy, Mercapto, Cyano, Isocyanato, Nitro, Epoxy, $SO_3H$ oder $PO_4H_2$. Unter den Halogenen sind Fluor, Chlor und Brom und insbesondere Chlor bevorzugt.

In besonderen Ausführungsformen der erfindungsgemäßen Membranen werden Silane der allgemeinen Formel IV' eingesetzt.

$$[(HS-R^5)_nR^6-S-E-R^5]_aSiX_xR_{4-a-x} \qquad (IV')$$

in der die Reste und Indices folgende Bedeutung haben:

E = -CO-NH-, -CS-NH-, $-CH_2-CH_2-$ oder $-CH_2-CH(OH)-$

R = wie im Falle der allgemeinen Formel IV definiert;

$R^5$ = Alkylen, Arylen, Arylenalkylen oder Arylenalkylen mit jeweils 1 bis 15 Kohlenstoff-Atomen, wobei diese Reste durch Sauerstoff- und/oder durch Schwefel-Atome und/oder durch Ester- und/oder durch Carbonyl- und/oder durch Carboxyl- und/oder durch Amid- und/oder durch Amino-Gruppen unterbrochen sein können;
$R^6$ = Alkylen, Arylen, Arylenalkylen oder Arylenalkylen mit jeweils 1 bis 15 Kohlenstoff-Atomen, wobei diese Reste durch Sauerstoff- und/oder durch Schwefel-Atome und/oder durch Ester- und/oder durch Carbonyl- und/oder durch Carboxyl- und/oder durch Amid- und/oder durch Amino-Gruppen unterbrochen sein können;
X = wie im Falle der allgemeinen Formel IV definiert;
a = wie im Falle der allgemeinen Formel IV definiert;
n = 2, 3, 4 oder 5;
x = wie im Falle der allgemeinen Formel IV definiert.

Organisch modifizierte Silane der allgemeinen Formel IV', deren Herstellung sowie konkrete Beispiele sind in der DE 19627220 C2 ausführlichst beschrieben. Auf die Offenbarung der DE 19627220 C2 wird hier ausdrücklich Bezug genommen.

In der allgemeinen Formel V haben die Reste und Indices folgende Bedeutung, wobei bei Indices ≥ 2 die diesbezüglichen Reste gleich oder verschieden sind.

$$X_aSiR_{4-a} \qquad (V)$$

R = Alkyl, Alkenyl, Aryl, Alkylaryl oder Arylalkyl mit jeweils 1 bis 15 Kohlenstoff-Atomen, wobei diese Reste Sauerstoff- und/oder Schwefel-Atome und/oder Ester- und/oder Carbonyl- und/oder Carboxyl- und/oder Amid- und/oder Amino-Gruppen enthalten können.
X = Wasserstoff, Halogen, Hydroxy, Alkoxy, Acyloxy, Alkylcarbonyl, Alkoxycarbonyl oder $NR''_2$, mit R'' = Wasserstoff, Alkyl, Aryl oder Alkylaryl.
a = 1, 2 oder 3.

Die Alkyl-Reste sind z.B. geradkettige, verzweigte oder cyclische Reste mit 1 bis 20, insbesondere mit 1 bis 10 Kohlenstoff-Atomen und vorzugsweise niedere Alkyl-Reste mit 1 bis 6, besonders bevorzugt mit 1 bis 4 Kohlenstoff-Atomen. Spezielle Beispiele sind Methyl, Ethyl, n-Propyl, i-Propyl, n- Butyl, i-Butyl, s-Butyl, t-Butyl, n-Pentyl, n-Hexyl, Cyclohexyl, 2-Ethylhexyl, Dodecyl und Octadecyl. Die Alkenyl-Reste sind z.B. geradkettige, verzweigte oder cyclische Reste mit 2 bis 20, bevorzugt mit 2 bis 10 Kohlenstoff-Atomen und vorzugsweise niedere Alkenyl-Reste mit 2 bis 6 Kohlenstoff-Atomen, wie z.B. Vinyl, Allyl und 2-Butenyl. Bevorzugte Aryl-Reste sind Phenyl, Biphenyl und Naphthyl.

Die Alkoxy-, Acyloxy-, Alkylamino-, Dialkylamino-, Alkylcarbonyl-, Alkoxycarbonyl-, Arylaklyl-, Alkylaryl-, Alkylen- und Alkylenarylen-Reste leiten sich vorzugsweise von den oben genannten Alkyl- und Aryl-Resten ab. Spezielle Beispiele sind Methoxy, Ethoxy, n- und i-Propoxy, n-, i-, s- und t- Butoxy, Monomethylamino, Monoethylamino, Dimethylamino, Diethylamino, N-Ethylanilino, Acetyloxy, Propionyloxy, Methylcarbonyl, Ethylcarbonyl, Methoxycarbonyl, Ethoxycarbonyl, Benzyl, 2-Phenylethyl und Tolyl.

Die genannten Reste können gegebenenfalls einen oder mehrere Substituenten tragen, z.B. Halogen, Alkyl, Hydroxyalkyl, Alkoxy, Aryl, Aryloxy, Alkylcarbonyl, Alkoxycarbonyl, Furfuryl, Tetrahydrofurfuryl, Amino, Monoalkylamino, Dialkylamino, Trialkylammonium, Amido, Hydroxy, Formyl, Carboxy, Mercapto, Cyano, Isocyanato, Nitro, Epoxy, $SO_3H$ oder $PO_4H_2$. Unter den Halogenen sind Fluor, Chlor und Brom und insbesondere Chlor bevorzugt.

Silane der allgemeinen Formel V sind entweder im Handel erhältlich oder lassen sich nach bekannten Methoden herstellen, die z.B. in "Chemie und Technologie der Silicone", W. Noll, Verlag Chemie GmbH, Weinheim/Bergstraße (1968), beschrieben sind.

Ohne Einschränkung der Allgemeinheit sind konkrete Beispiele für Silane der allgemeinen Formel V: $CH_3$-Si-$Cl_3$, $CH_3$-Si-$(OC_2H_5)_3$, $C_2H_5$-Si-$Cl_3$, $C_2H_5$-Si-$(OC_2H_5)_3$, $CH_2$=CH-Si-$(OC_2H_5)_3$, $CH_2$=CH-Si-$(OC_2H_4OCH_3)_3$, $(CH_3)_2$-Si-$Cl_2$, $CH_2$=CH-Si-$(OOCCH_3)_3$, $(CH_3)_2$-Si-$(OC_2H_5)_2$, $(C_2H_5)_3$-Si-Cl, $(C_2H_5)_2$-Si-$(OC_2H_5)_2$. $(CH_3)_2(CH_2$=CH)-Si-$Cl_2$, $(CH_3)_3$-Si-Cl, $(t-C_4H_9)(CH_3)_2$-Si-Cl, $(CH_3O)_3$-Si-$C_3H_6$-NH-$C_2H_4$-NH-$C_2H_4$-$NH_2$, $(CH_3O)_3$-Si-$C_3H_6$-SH, $(CH_3O)_3$-Si-$C_3H_6$-NH-$C_2H_4$-$NH_2$, $(CH_3O)_3$-Si-$C_3H_6$-Cl, $(CH_3)_2(CH_2$=CH-$CH_2)$-Si-Cl, $(CH_3O)_3$-Si-$C_3H_6$-O-C(O)-C$(CH_3)$=$CH_2$, $(C_2H_5O)_3$-Si-$C_3H_6$-$NH_2$, $(C_2H_5O)_3$-Si-$C_3H_6$-CN·

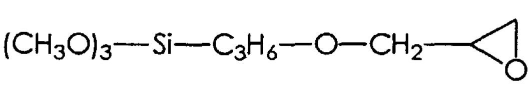 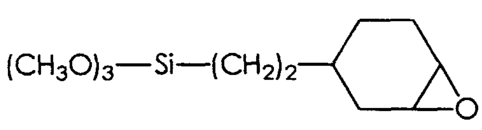

Die Silane der allgemeinen Formeln I, II, III, IV, IV' und V sind über die Reste X hydrolysierbar und polykondensierbar, und durch die hydrolytische Polykondensation wird ein anorganisches Netzwerk mit Si-O-Si-Brücken aufgebaut. Die Polykondensation erfolgt vorzugsweise nach dem Sol-Gel-Verfahren, wie es z.B. in den DE-A1 2758414, 2758415, 3011761, 3826715 und 3835968 beschrieben ist. Die Polykondensation wird in der auf diesem Gebiet üblichen Art und Weise durchgeführt, indem man z.B. den zu hydrolysierenden Silicium-Verbindungen, die entweder als solche oder gelöst in einem Lösungsmittel vorliegen, das erforderliche Wasser bei Raumtemperatur oder unter leichter Kühlung direkt zugibt (vorzugsweise unter Rühren und in Anwesenheit eines Hydrolyse- und Kondensationskatalysators ) und die resultierende Mischung daraufhin einige Zeit (ein bis mehrere Stunden) rührt. Bei Anwesenheit reaktiver Verbindungen empfiehlt sich in der Regel eine stufenweise Zugabe des Wassers. Unabhängig von der Reaktivität der anwesenden Verbindungen erfolgt die Hydrolyse in der Regel bei Temperaturen zwischen -20 und 130 °C bzw. dem Siedepunkt des gegebenenfalls eingesetzten Lösungsmittels. Wie bereits angedeutet, hängt die beste Art und Weise der Zugabe von Wasser vor allem von der Reaktivität der eingesetzten Ausgangsverbindungen ab. So kann man z.B. die gelösten Ausgangsverbindungen langsam zu einem Überschuss an Wasser tropfen oder man gibt Wasser in einer Portion oder portionsweise den gegebenenfalls gelösten Ausgangsverbindungen zu. Es kann auch nützlich sein, das Wasser nicht als solches zuzugeben, sondern mit Hilfe von wasserhaltigen organischen oder anorganischen Systemen in das Reaktionssystem einzutragen. Als besonders geeignet hat sich in vielen Fällen die Eintragung der Wassermenge in das Reaktionsgemisch mit Hilfe von Feuchtigkeits beladenen Adsorbentien, z.B. von Molekularsieben, und von wasserhaltigen, organischen Lösungsmitteln, z.B. von 80 %-igem Ethanol, erwiesen. Die Wasserzugabe kann aber auch über eine chemische Reaktion erfolgen, bei der Wasser im Laufe der Reaktion freigesetzt wird. Beispiele hierfür sind Veresterungen.

Wenn ein Lösungsmittel verwendet wird, kommen neben den niederen aliphatischen Alkoholen (z.B. Ethanol oder i-Propanol) auch Ketone, vorzugsweise niedere Dialkylketone, wie Aceton oder Methylisobutylketon, Ether, vorzugsweise niedere Dialkylether wie Diethylether oder Dibutylether, THF, Amide, Ester, insbesondere Essigsäureethylester, Dimethylformamid, Amine, insbesondere Triethylamin, und deren Gemische in Frage.

Die Ausgangsverbindungen müssen nicht notwendigerweise bereits alle zu Beginn der Hydrolyse (Polykondensation) vorhanden sein, sondern es kann sich sogar als vorteilhaft erweisen, wenn nur ein Teil dieser Verbindungen zunächst mit Wasser in Kontakt gebracht wird und später die restlichen Verbindungen zugegeben werden.

Um Ausfällungen während der Hydrolyse und der Polykondensation so weit wie möglich zu vermeiden, kann die Wasserzugabe in mehreren Stufen, z.B. in drei Stufen, durchgeführt werden. Dabei kann in der ersten Stufe z.B. ein Zehntel bis ein Zwanzigstel der zur Hydrolyse benötigten Wassermenge zugegeben werden. Nach kurzem Rühren kann die Zugabe von einem Fünftel bis zu einem Zehntel der erforderlichen Wassermenge erfolgen und nach weiterem kurzen Rühren kann schließlich der Rest zugegeben werden.

Die Kondensationszeit richtet sich nach den jeweiligen Ausgangskomponenten und deren Mengenanteilen, dem gegebenenfalls verwendeten Katalysator, der Reaktionstemperatur, etc. Im allgemeinen erfolgt die Polykondensation bei Normaldruck, sie kann jedoch auch bei erhöhtem oder bei verringertem Druck durchgeführt werden.

Das so erhaltene Polykondensat kann entweder als solches oder nach teilweiser oder nahezu vollständiger Entfernung des verwendeten Lösungsmittels zu den erfindungsgemäßen Membranen verarbeitet werden. In einigen Fällen kann es sich als vorteilhaft erweisen, in dem nach der Polykondensation erhaltenen Produkt das überschüssige Wasser und das gebildete und gegebenenfalls zusätzlich eingesetzte Lösungsmittel durch ein anderes Lösungsmittel zu ersetzen, um das Polykondensat zu stabilisieren. Zu diesem Zweck kann die Reaktionsmischung z.B. im Vakuum bei leicht erhöhter Temperatur so weit eingedickt werden, dass sie noch problemlos mit einem anderen Lösungsmittel aufgenommen werden kann.

Das auf diese Weise erhaltene Polykondensat stellt eine mehr oder weniger viskose Flüssigkeit oder ein Harz dar, und es wird nach üblichen Methoden zu Flachmembranen oder zu rohrförmigen Membranen verarbeitet. Nach der Formgebung und einer gegebenenfalls erforderlichen Trocknung wird die resultierende Membran durch Ausbildung eines organischen Netzwerkes gehärtet.

Die Silane der Formel I und daraus resultierende Polykondensate können über die bicyclischen Reste, die Silane der Formeln II und III und deren Polykondensate über die Reste B und die Silane der Formeln IV und IV' und deren Polykondensate über die Mercapto-Gruppen einer Polymerisation und/oder einer Polyaddition unterzogen werden. Durch diese Polymerisations- bzw. Polyadditionsreaktionen wird ein organisches Netzwerk aufgebaut. Das aus den Silanen der Formeln I bis V resultierende Polykondensat bzw. die daraus gefertigte Membran kann somit durch Polymerisation und/oder durch Polyaddition gehärtet werden. Diese Härtungsreaktionen werden thermisch und/oder strahlungsinduziert und/oder chemisch induziert durchgeführt. Nach der Härtung resultiert ein anorganisch-organisches Netzwerk, d.h. die erfindungsgemäßen Membranen verfügen über ein anorganisch-organisches Netzwerk. Durch Variation des anorganischen und/oder des organischen Netzwerkes, z.B. der Netzwerksdichte, können die chemischen und physikalischen Eigenschaften der erfindungsgemäßen Membranen in weiten Bereichen variiert und das Eigenschaftsprofil der erfindungsgemäßen Membranen kann den Anforderungen des jeweiligen Anwendungsfalles angepasst werden.

Das für die Herstellung der erfindungsgemäßen Membranen eingesetzte Polykondensat kann weitere Zusatzstoffe enthalten. Diese Zusatzstoffe können vor und/oder während und/oder nach der Polykondensation zugegeben werden. Bei diesen Zusatzstoffen handelt es sich z.B. um copolymerisierbare und/oder addierbare und/oder polyaddierbare Monomere und/oder Oligomere. Diese Monomere bzw. Oligomere werden im Zuge der Härtung der resultierenden Membran über Polymerisations- und/oder (Poly)Additionsreaktionen in das organische Netzwerk der erfindungsgemäßen Membran eingebaut. Werden hydrolysierbare Silicium-Verbindungen mit SH- bzw.- C=C- bzw. Amino-Gruppen eingesetzt und werden diese vor der hydrolytischen Polykondensation zugegeben, so werden diese Verbindungen im Zuge der Polykondensation in das anorganische und im Zuge der Polymerisation bzw. (Poly)Addition in das organische Netzwerk der erfindungsgemäßen Membranen eingebaut.

Weitere Zusatzstoffe, die das für die Herstellung der erfindungsgemäßen Membranen eingesetzte Polykondensat enthalten kann, sind z.B. Härtungskatalysatoren. Diese werden z.B. erforderlich, wenn die resultierende Membran chemisch induziert gehärtet wird.

Die erfindungsgemäßen Oxygenatormembranen sind nicht porös.

Zur Herstellung von z.B. Endlosflachmembranen werden bevorzugt Lösungsmittel freie Systeme eingesetzt, - lösungsmittelhaltige sind jedoch ebenfalls verarbeitbar, - die kontinuierlich auf eine rotierende Walze aufgetragen werden. Nach der Filmbildung mittels Spaltrakel erfolgt die Härtung, z.B. eine strahlungsinduzierte Härtung, die Ablösung und Aufwickelung der Membranen.

Zur Herstellung von z.B. Endloshohlfasern werden bevorzugt lösungsmittelfreie Systeme eingesetzt, - lösungsmittelhaltige sind jedoch ebenfalls verarbeitbar, - aus denen wie folgt Hohlfasern gefertigt werden. Zunächst wird das harzförmige Polykondensat durch eine ringförmige Düse extrudiert, wobei der Hohlraum durch eine Gas bzw. Flüssigkeit führende Innendüse erzeugt wird. Die Dimension des Harzfadens wird nach üblichen Methoden durch die Variation der Spinnparameter, wie z.B. der Abzugsgeschwindigkeit, der Temperatur, des Drucks, etc., eingestellt. Anschließend wird durch eine ringförmige Vorhärtungskomponente, z.B. eine Strahlungsquelle, unmittelbar unterhalb der Spinndüse der Harzfaden vorvernetzt und damit die Form konserviert. Mittels eines darunter angebrachten Rundstrahlers wird die Endhärtung durchgeführt. Die resultierende Endlosfaser wird aufgewickelt und umgespult. Zur Vorhärtung und/oder zur Endhärtung kann neben einer strahlungsinduzierten Härtung auch eine selbstinduzierte oder eine chemisch induziere Härtung durchgeführt werden. Eine Kombination verschiedener Härtungsprinzipien ist ebenfalls möglich.

Die Härtung der Membranen erfolgt nach üblichen Methoden thermisch, Strahlungs oder chemisch induziert. Gegebenenfalls ist die Zugabe von üblichen Härtungskatalysatoren erforderlich. Die Härtung erfolgt nach Methoden, wie sie z.B. in der DE 4011044 C2, DE 4310733 A1, DE 4405261 A1, DE 4416857 C1, DE 19627198 C2 und der DE 19627220 C2 beschrieben sind.

Die erfindungsgemäßen Membranen sind in einphasiger wie auch in zweiphasiger Ausführung herstellbar. Zweiphasige Membranen werden aus Systemen erhalten, die nicht mischbar sind, sondern Emulsionen bilden. Derartige Systeme können sowohl zu Fasern als auch zu Folien verarbeitet werden, indem man z.B. durch gemeinsames Rühren eine Emulsion der nicht mischbaren Komponenten fertigt, diese Emulsion nach üblichen Methoden zu Membranen verarbeitet und bei der Härtung der resultierenden Membran die nicht-mischbaren Komponenten zusammen härtet. Eine andere Variante besteht darin, Systeme zu verarbeiten, bei denen es während der Synthese zur Phasentrennung kommt. Durch diese zweiphasigen Varianten lassen sich erfindungsgemäße Membranen fertigen, die aus einem stabilen organisch-anorganischen Netzwerk bestehen, in das eine durchgehende, hochpermeable zweite Phase eingelagert ist.

Das Herstellungsverfahren der erfindungsgemäßen Membranen ist einfach, kostengünstig und auf kleinstem Raum durchführbar. Es ist für die Endlosproduktion von Hohlfasern und Folien geeignet und es sind alle gängigen Härtungsprinzipien anwendbar. Aufgrund der toxikologischen Unbedenklichkeit der Materialien sind die erfindungsgemäßen Membranen im medizinischen Bereich problemlos anwendbar. Die für verschiedene Anwendungen oftmals notwendigen Oberflächen-Modifikationen, z.B. zur Vermeidung von Blutkoagulationen, zur Einstellung der Polarität, des Adsorptionsverhaltens etc., können entweder schon während der Materialsynthese, d.h. in situ, oder nachträglich durchgeführt werden. Derartige Oberflächenmodifikationen sind z.B. Beschichtungen mit Heparin, mit hydrophilen oder hydrophoben Silanen, mit Fluorsilanen oder mit Biomolekülen.

Die erfindungsgemäßen Membranen zeigen bei hoher mechanischer Stabilität sehr gute Permeationswerte, auch ohne Porosität. Damit sind auch bei hohen Permeationswerten noch freitragende Folien und Hohlfasern herstellbar, ohne dass z.B. die Gefahr des Durchtritts der fluiden Phase besteht.

Durch folgende beispielhafte Modifikationen kann die Permeabilität der Membran den Anforderungen des jeweiligen Anwendungsfalles angepasst werden.

- Variation der anorganischen und der organischen Strukturdichte
- Variation des Gehalts an Dimethylsilan-Einheiten
- Chemischer und physikalischer Einbau von anorganischen oder organischen vorgefertigten, hochpermeablen Monomeren, Oligomeren oder Polymeren

- Silanisierung freier SiOH-Gruppen mit Trimethylsilyl-Einheiten

Variation der anorganischen Strukturdichte

Ohne Einschränkung der Allgemeinheit wird am Vergleich der folgenden Harzsysteme der Einfluss der anorganischen Strukturdichte auf $O_2$-Permeabilität, E-Modul und Festigkeit der resultierenden Membranen aufgezeigt. Die Ergebnisse dazu sind tabellarisch zusammengefasst. Ganz allgemein kann gesagt werden, dass eine Erhöhung der anorganischen Strukturdichte zu einer Erhöhung der mechanischen Stabilität und zu einer Erniedrigung der Sauerstoffpermeabilität führt.

| Harztyp | Anzahl d. hydrolysier- und kondensierbaren Gruppen pro Basiseinheit. | $O_2$-Permeabilität [x] | E-Modul [MPa] | Festigkeit [MPa] |
|---|---|---|---|---|
| 1 | 3 | $0.09 \cdot 10^{-10}$ | 2640 | 106 |
| 2 | 2 | $0.23 \cdot 10^{-10}$ | 1520 | 59 |
| 3 | 4 | $0.07 \cdot 10^{-10}$ | 3000 | 120 |

$O_2$-Permeabilität : x = $cm^3/cm \cdot s \cdot cmHg$

| Harztyp | Anzahl d. hydrolysier- und kondensierbaren Gruppen pro Basiseinheit | $O_2$-Permeabilität [$cm^3/cm \cdot s \cdot cmHg$] |
|---|---|---|
| 4 | 2 | $11.5 \cdot 10^{-10}$ |
| 5 | 2.5 | $3.9 \cdot 10^{-10}$ |
| 6 | 3 | $1.2 \cdot 10^{-10}$ |

| | Edukte | molares Verhältnis |
|---|---|---|
| Harztyp 1: | Glycerin-1,3-dimethacrylat | 1.0 |
| | 3-Isocyanatopropyltriethoxysilan | 1.0 |
| | 1,12-Dodecandioldimethacrylat | 0.2 |
| Harztyp 2: | Trimethylolpropantriacrylat | 1.2 |
| | Mercaptopropylmethyldimethoxysilan | 1.0 |
| Harztyp 3: | Tris(2-Hydroxyethyl)isocyanurat-triacrylat | 1.0 |
| | Mercaptopropylmethyldimethoxysilan | 1.0 |
| | Tetraethoxysilan | 1.0 |
| Harztyp 4: | Trimethylolpropantriacrylat | 1.0 |
| | Mercaptopropylmethyldimethoxysilan | 1.0 |
| | Dimethyldiethoxysilan | 4.0 |
| Harztyp 5: | Trimethylolpropantriacrylat | 1.0 |

(fortgesetzt)

| | Edukte | molares Verhältnis |
|---|---|---|
| Harztyp 6: | Mercaptopropylmethyldimethoxysilan | 1.0 |
| | Dimethyldiethoxysilan | 2.0 |
| | Methyltrimethoxysilan | 2.0 |
| | Trimethylolpropantriacrylat | 1.0 |
| | Mercaptopropylmethyldimethoxysilan | 1.0 |
| | Methyltrimethoxysilan | 4.0 |

Beim Harztyp 1 werden zunächst, gemäß folgendem Reaktionsschema, Glycerin-1,3-dimethacrylat und 3-Isocyanatopropyltriethoxysilan miteinander verknüpft.

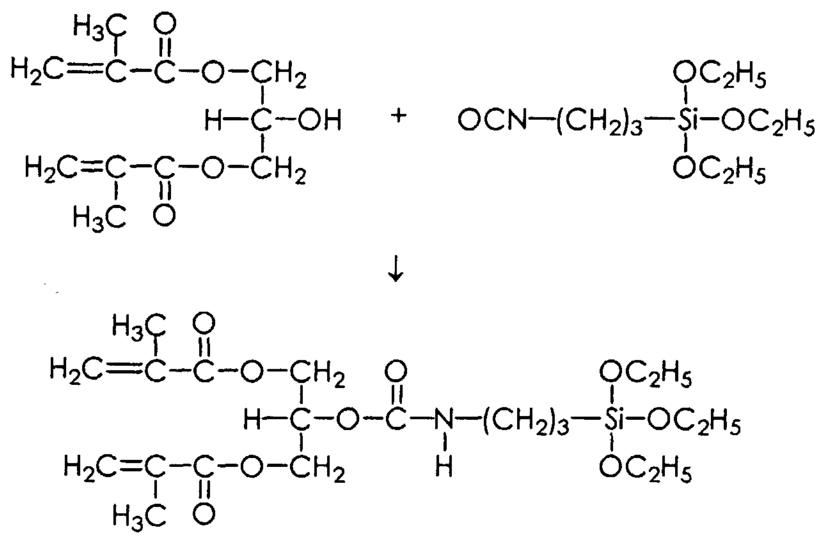

Das resultierende Silan wird zum Aufbau des anorganischen Netzwerkes hydrolytisch polykondensiert, wobei das 1,12-Dodecandioldimethacrylat vor, während oder nach der Polykondensation zugegeben werden kann. Aus der resultierenden Mischung werden nach üblichen Verfahren Membranen gefertigt, bei deren Härtung durch Polymerisation der Methacrylat-Gruppen das organische Netzwerk aufgebaut wird.

Beim Harztyp 2 werden zunächst, gemäß folgendem Reaktionsschema, das Trimethylolpropantriacrylat und das Mercaptopropylmethyldimethoxysilan miteinander verknüpft.

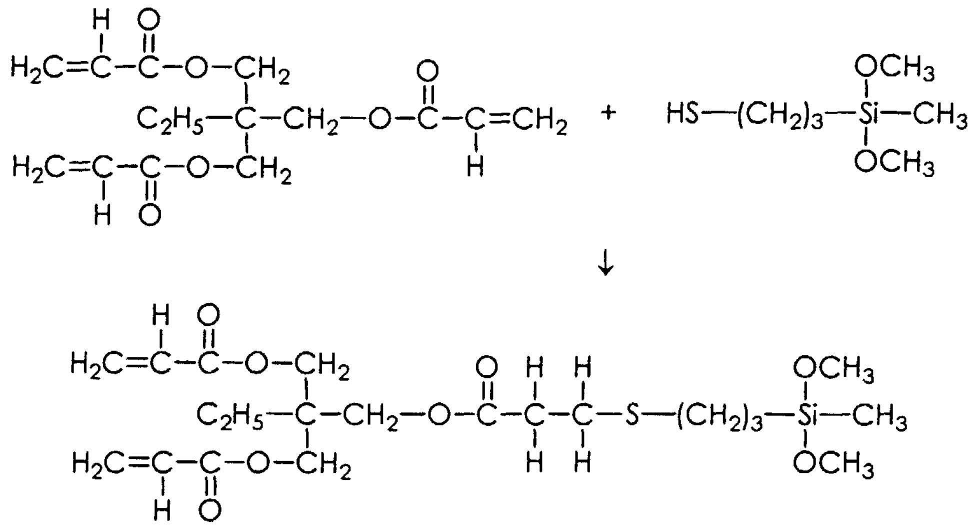

Das resultierende Silan wird zum Aufbau des anorganischen Netzwerkes hydrolytisch polykondensiert. Aus dem Polykondensat werden dann nach üblichen Verfahren Membranen gefertigt, bei deren Härtung durch Polymerisation der Acrylat-Gruppen das organische Netzwerke aufgebaut wird.

Beim Harztyp 3 werden zunächst, gemäß folgendem Reaktionsschema, das Tris(2-Hydroxyethyl)isocyanurat-triacrylat und das Mercaptopropylmethyldimethoxysilan miteinander verknüpft.

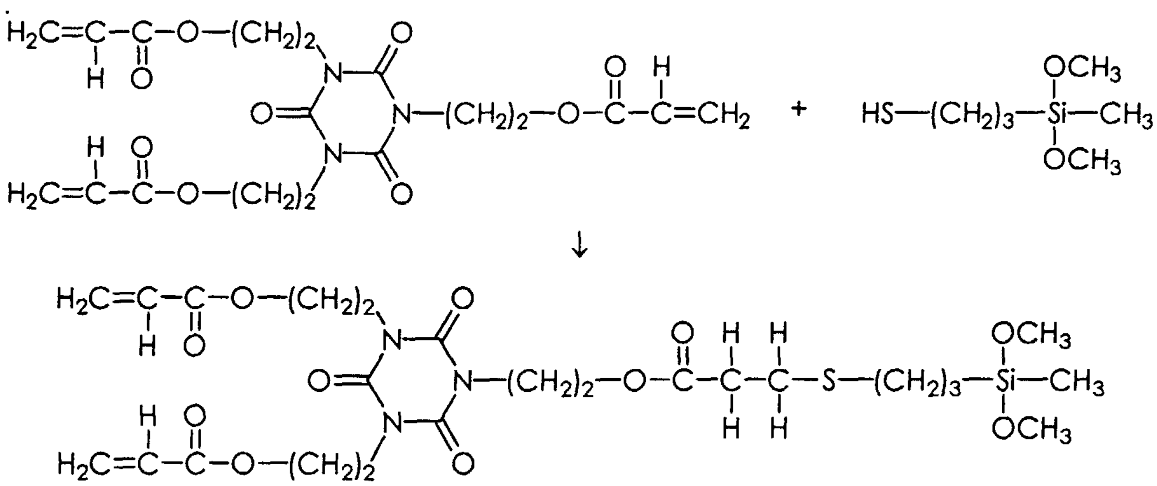

Anschließend wird zum Aufbau des anorganischen Netzwerkes das resultierende Silan, zusammen mit dem Tetraethoxysilan, hydrolytisch polykondensiert, und die daraus gefertigte Membran wird durch Polymerisation der Acrylat-Gruppen gehärtet.

Beim Harztyp 4 werden zunächst, in Analogie zum Harztyp 2, das Trimethylolpropantriacrylat und das Mercaptopropylmethyldimethoxysilan miteinander verknüpft. Anschließend wird das resultierende Silan, zusammen mit dem Dimethyldiethoxysilan, hydrolytisch polykondensiert, und die daraus gefertigte Membran wird durch Polymerisation der Acrylat-Gruppen gehärtet.

Beim Harztyp 5 werden zunächst, in Analogie zum Harztyp 2, das Trimethylolpropantriacrylat und das Mercaptopropylmethyldimethoxysilan miteinander verknüpft. Anschließend wird das resultierende Silan, zusammen mit dem Dimethyldiethoxysilan und dem Methyltrimethoxysilan, hydrolytisch polykondensiert, und die daraus gefertigte Membran wird durch Polymerisation der Acrylat-Gruppen gehärtet.

Beim Harztyp 6 werden zunächst, in Analogie zum Harztyp 2, das Trimethylolpropantriacrylat und das Mercaptopropylmethyldimethoxysilan miteinander verknüpft. Anschließend wird das resultierende Silan, zusammen mit dem Methyltrimethoxysilan, hydrolytisch polykondensiert, und die daraus gefertigte Membran wird durch Polymerisation der Acrylat-Gruppen gehärtet.

Bei gleichem organischen Vernetzungspotential ergibt sich wegen des höheren anorganischen Vernetzungspotentials von System 1 ein gegenüber System 2 höherer E-Modul, eine höhere Biegebruchfestigkeit und ein erniedrigter $O_2$-Permeationskoeffizient. Die Zugabe des vierfach hydrolysier- und kondensierbaren Tetraethoxysilan bewirkt bei System 3 gegenüber System 2 eine Erhöhung der anorganischen Vernetzungsdichte bei gleichzeitiger Reduzierung der $O_2$-Permeabilität. Der Vergleich der Systeme 4, 5 und 6 zeigt, dass der Ersatz von Methylgruppen durch das Vernetzungspotential erhöhenden Alkoxygruppen zu einer Reduzierung der $O_2$-Permeabilität führt.

Variation der organischen Strukturdichte, Silanisierung von SiOH-Gruppen und Einbau von permeationssteigernden Monomeren

Ohne Einschränkung der Allgemeinheit wird am Vergleich von weiteren Harzsystemen der Einfluss der organischen Strukturdichte auf $O_2$-Permeabilität, E-Modul und Festigkeit der resultierenden Membranen aufgezeigt. Die Ergebnisse dazu sind tabellarisch zusammengefasst. Ganz allgemein kann gesagt werden, dass eine Verringerung des organischen Vernetzungspotentials eine deutliche Reduzierung der mechanischen Festigkeit und eine Erhöhung der $O_2$-Permeabilität bewirkt. Durch die Silanisierung von SiOH-Gruppen und/oder durch den Einbau von Reaktivmonomeren kann die $O_2$-Permeabilität der erfindungsgemäßen Membranen weiter erhöht werden.

Die Polykondensate sind in der Regel nicht vollständig anorganisch kondensiert, d.h. es sind freie ≡SiOH-Gruppen vorhanden. Diese können im Rahmen einer Silanisierung z.B. zu $\equiv Si\text{-}O\text{-}Si(CH_3)_3$-Gruppen umgesetzt werden. Dies bewirkt zum einen eine Auflockerung der Gesamtstruktur der erfindungsgemäßen Membran und zum anderen eine Erhöhung der Zahl der die $O_2$-Permeabilität begünstigenden Si-O-Si-Gruppen. Kontrolliert wird die Silanisierung durch Aufnahme des IR-Spektrums anhand des Verschwindens der verbliebenen SiOH-Bande.

| | Edukte | molares Verhältnis |
|---|---|---|
| Harztyp 2: | Trimethylolpropantriacrylat | 1.2 |
| | Mercaptopropylmethyldimethoxysilan | 1.0 |
| Harztyp 7: | 1.12-Dodecandioldimethacrylat | 1.0 |
| | Mercaptopropylmethyldimethoxysilan | 1.0 |
| Harztyp 8: | Silanisierter Harztyp 7 | |
| Harztyp 9: | Silanisierter Harztyp 2 | 2.0 |
| | Methacryloxypropyltris(trimethylsiloxy) silan (TRIS) | 1.0 |
| Harztyp 10: | Silanisierter Harztyp 7 | 3.0 |
| | 1,3-Bis(3-methacryloxypropyl)tetrakis(trimethylsiloxy)disiloxan (TETRAKIS) | 1.0 |

| Harz-typ | Anzahl d. org. vernetzbaren Gruppen pro Basiseinheit | $O_2$-Permeabilität [ x ] | E-Modul [MPa] |
|---|---|---|---|
| 2 | 2 | 0.23 $10^{-10}$ | 1520 |
| 7 | 1 | 3.2 $10^{-10}$ | 11.2 |

$O_2$-Permeabilität : x = $cm^3/cm \cdot s \cdot cmHg$

| Harztyp | $O_2$-Permeabilität [ $cm^3/cm \cdot s \cdot cmHg$ ] |
|---|---|
| 2 silanisiert | 2.3 $10^{-10}$ |
| 8 | 13.9 $10^{-10}$ |
| 9 | 13.2 $10^{-10}$ |
| 10 | 20 $10^{-10}$ |

Die Herstellung von Membranen aus dem Harztyp 2 ist bei der Diskussion der anorganischen Strukturdichte beschrieben.

Beim Harztyp 7 werden zunächst, gemäß dem folgenden Reaktionsschema, das 1.12-Dodecandioldimethacrylat und das Mercaptopropylmethyldimethoxysilan mit einander verknüpft.

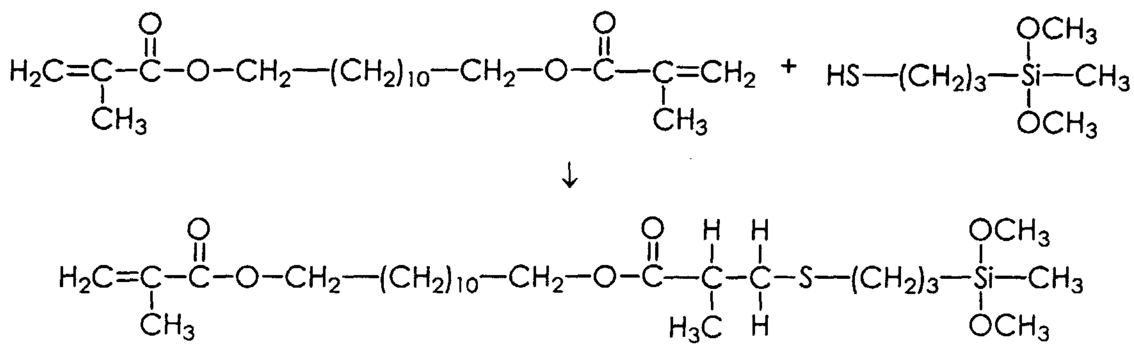

Anschließend wird das resultierende Silan hydrolytisch polykondensiert, und die daraus gefertigte Membran wird durch Polymerisation der Methacrylat-Gruppen gehärtet.

Beim Harztyp 8 werden zunächst, gemäß dem folgenden Reaktionsschema, nach der Polykondensation noch vorhandene SiOH-Gruppen des Harztyp 7 silanisiert.

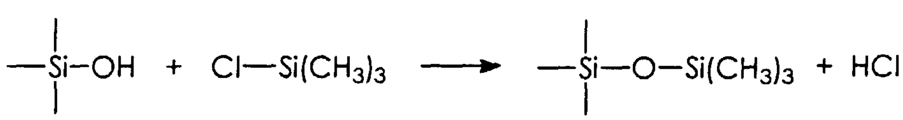

Die daraus gefertigten Membranen werden durch Polymerisation der Methacrylat-Gruppen gehärtet.

Bei den Harztypen 9 und 10 werden nach der Silanisierung die Reaktivmonomere TRIS bzw. TETRAKIS eingearbeitet.

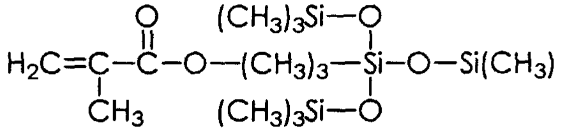

TRIS

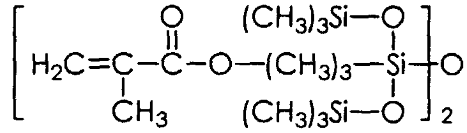

TETRAKIS

Die daraus gefertigten Membranen werden durch Polymerisation der Methoxy-Gruppen gehärtet.

Bei gleicher anorganischer Vernetzung der Systeme 2 und 7 bewirkt eine Verringerung des organischen Vernetzungspotentials von System 7 eine deutliche Reduktion des E-Moduls und eine Erhöhung der Permeation. Harztyp

8 zeigt, im Vergleich zu Harztyp 7, als Folge der Silanisierung eine weitere Erhöhung der $O_2$-Permeabilität. Im Vergleich zum Harztyp 8 wird beim Harztyp 9 durch die Zugabe des Reaktivmonomers TRIS der Einbau von die $O_2$-Permeation begünstigenden Endgruppen in die resultierende Membran bewirkt. Im Vergleich zum Harztyp 8 wird beim Harztyp 10 durch die Zugabe der Vernetzungskomponente TETRAKIS die $O_2$-Permeabilität der resultierenden Membran weiter erhöht.

Einbau von Dimethylsiloxan-Strukturen

Durch den Einbau von Dimethylsiloxan-Strukturen wird die $O_2$-Permeabilität der resultierenden Membran ebenfalls erhöht. Ohne Einschränkung der Allgemeinheit erfolgt der Einbau in das Polykondensat z.B. durch Co-Kondensation von z.B. Dimethyldialkoxysilan, durch Addition von Amino terminierten Polydimethylsiloxan oder durch Co-Polymerisation von Acryloxy terminiertem Polydimethylsiloxan. Der daraus resultierende Variationsbereich der $O_2$-Permeation beträgt drei Größenordnungen.

Bei den folgenden Beispielen, deren Ergebnisse tabellarisch zusammengefasst sind, erfolgt der Einbau der Dimethylsiloxan-Strukturen in das Polykondensat durch Co-Polymerisation.

| Harztyp | Zahl d. $-Si(CH_3)_2-O$-Gruppen/Basiseinheit | $O_2$-Permeabilität [ x ] |
|---|---|---|
| 11 | 0 | $0.09 \cdot 10^{-10}$ |
| 12 | 4 | $22 \cdot 10^{-10}$ |
| 13 | 6 | $66 \cdot 10^{-10}$ |
| 14 | 8 | $120 \cdot 10^{-10}$ |
| 15 | 10 | $140 \cdot 10^{-10}$ |

$O_2$-Permeabilität : $x = cm^3/cm \cdot s \cdot cmHg$

| | Edukte | molares Verhältnis |
|---|---|---|
| Harztyp 11: | Glycerin-1.3-dimethacrylat | 1 |
| | Isocyanatopropyltriethoxysilan | 1 |
| | Dimethyldiethoxysilan | 0 |
| Harztyp 12: | Glycerin-1.3-dimethacrylat | 1 |
| | Isocyanatopropyltriethoxysilan | 1 |
| | Dimethyldiethoxysilan | 4 |
| Harztyp 13: | Glycerin-1.3-dimethacrylat | 1 |
| | Isocyanatopropylfriethoxysilan | 1 |
| | Dimethyldiethoxysilan | 6 |
| Harztyp 14: | Glycerin-1.3-dimethacrylat | 1 |
| | Isocyanatopropyltriethoxysilan | 1 |
| | Dimethyldiethoxysilan | 8 |
| Harztyp 15: | Glycerin-1.3-dimethacrylat | 1 |
| | Isocyanatopropyltriethoxysilan | 1 |
| | Dimethyldiethoxysilan | 10 |

Bei den Harztypen 11 bis 15 werden zunächst, gemäß folgendem Reaktionsschema, Glycerin-1,3-dimethacrylat und 3-Isocyanatopropyltriethoxysilan miteinander verknüpft.

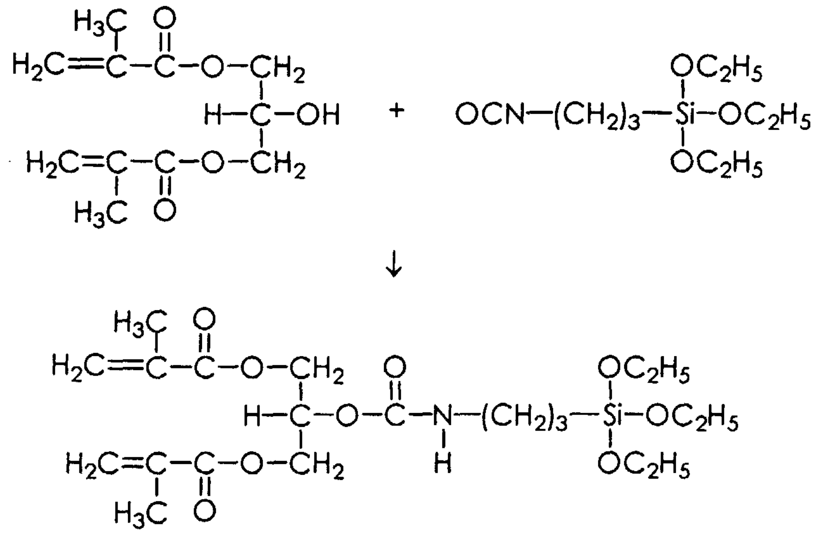

Das resultierende Silan wird dann zum Aufbau des anorganischen Netzwerkes entweder alleine (Harztyp 11) oder zusammen mit dem Dimethyldiethoxysilan (Harztypen 12 bis 15) hydrolytisch polykondensiert. Aus dem Polykondensat werden nach übliche Methoden Membranen gefertigt, bei deren Härtung durch Polymerisation der Methacrylat-Gruppen das organische Netzwerk aufgebaut wird.

Bei weiteren Ausführungsbeispielen erfolgt der Einbau von Dimethylsiloxan-Einheiten in das Polykondensat durch Co-Polykondensation mit Dimethyldiethoxysilan. Die Ergebnisse dazu sind tabellarisch zusammengefaßt.

| Harztyp | Zahl d. -$Si(CH_3)_2$-O-Gruppen/Basiseinheit | $O_2$-Permeabilität [ x ] |
|---|---|---|
| 16 | ½ | 1.7 $10^{-10}$ |
| 17 | 2 | 4.0 $10^{-10}$ |
| 18 | 4 | 9.1 $10^{-10}$ |
| 19 | 6 | 31 $10^{-10}$ |

$O_2$-Permeabilität : x = $cm^3/cm \cdot s \cdot cmHg$

| | Edukte | molares Verhältnis |
|---|---|---|
| Harztyp 16: | "Norbornen-Silan" | 1 |
| | Trimethylpropan-tris(3-mercaptopropionat | 1 |
| | Dimethyldiethoxysilan | ½ |
| Harztyp 17: | "Norbornen-Silan" | 1 |
| | Trimethylpropan-tris(3-mercaptopropionat | 1 |
| | Dimethyldiethoxysilan | 2 |
| Harztyp 18: | "Norbornen-Silan" | 1 |
| | Trimethylpropan-tris(3-mercaptopropionat | 1 |
| | Dimethyldiethoxysilan | 4 |
| Harztyp 19: | "Norbornen-Silan" | 1 |

(fortgesetzt)

| | Edukte | molares Verhältnis |
|---|---|---|
| | Trimethylpropan-tris(3-mercaptopropionat<br>Dimethyldiethoxysilan | 1<br>6 |

Zunächst wird das "Norbornen-Silan" der Harztypen 16 bis 19 gemäß dem folgenden Reaktionsschema hergestellt,

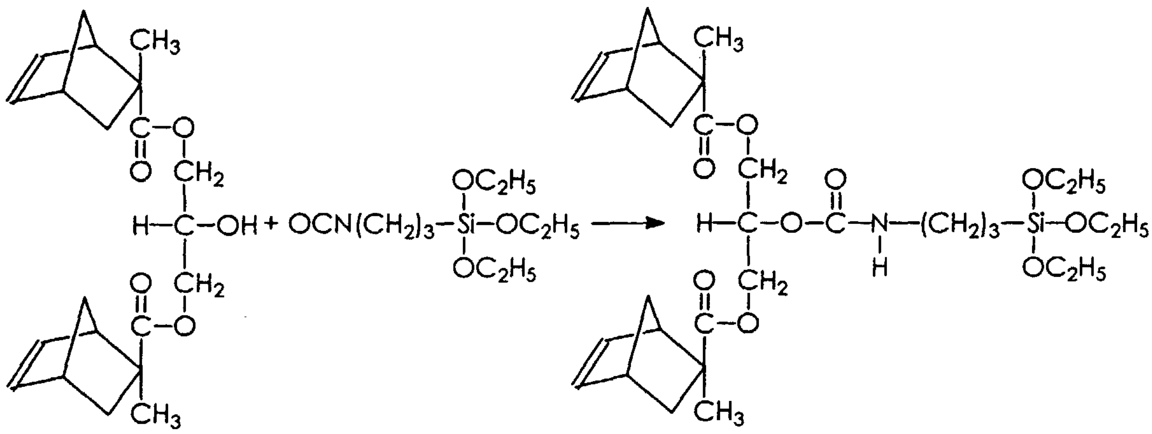

und anschließend mit dem Dimethyldiethoxysilan hydrolytisch polykondensiert. Das resultierende Polykondensat wird mit Trimethylpropan-tris(3-mercaptopropionat versetzt und zu Membranen verarbeitet, deren Härtung dann durch strahlungsinduzierte Polyaddition des Trimethylpropan-tris(3-mercaptopropionat an die C=C-Doppelbindungen der Norbornen-Reste erfolgt.

In den folgenden Beispielen erfolgt der Einbau der Dimethylsiloxan-Einheiten in das Polykondensat durch Addition von Aminopropyl terminiertem Polydimethylsiloxan mit ca. 65 -$Si(CH_3)_2$-O-Segmenten (= DMS A 21). Die Ergebnisse dazu sind tabellarisch zusammengefaßt.

| Harztyp | Zahl d. -$Si(CH_3)_2$-O-Gruppen/Basiseinheit | $O_2$-Permeabilität [ x ] |
|---|---|---|
| 20 | 2 | 19 $10^{-10}$ |
| 21 | 6 | 160 $10^{-10}$ |

$O_2$-Permeabilität : x = $cm^3/cm\cdot s\cdot cmHg$

| | Edukte | molares Verhältnis |
|---|---|---|
| Harztyp 20: | Trimethylolpropantriacrylat<br>Mercaptopropylmethyldimethoxysilan<br>DMS-A 21<br>Lösungsmittel : n-Butylacetat | 1.2<br>1.0<br>0.03<br> |
| Harztyp 21: | Trimethylolpropantriacrylat<br>Mercaptopropylmethyldimethoxysilan<br>DMS-A 21<br>Lösungsmittel : n-Butylacetat | 1.2<br>1.0<br>0.09<br> |

Beim Harztyp 20 und 21 werden zunächst, gemäß folgendem Reaktionsschema, das Trimethylolpropantriacrylat und das Mercaptopropylmethyldimethoxysilan miteinander verknüpft.

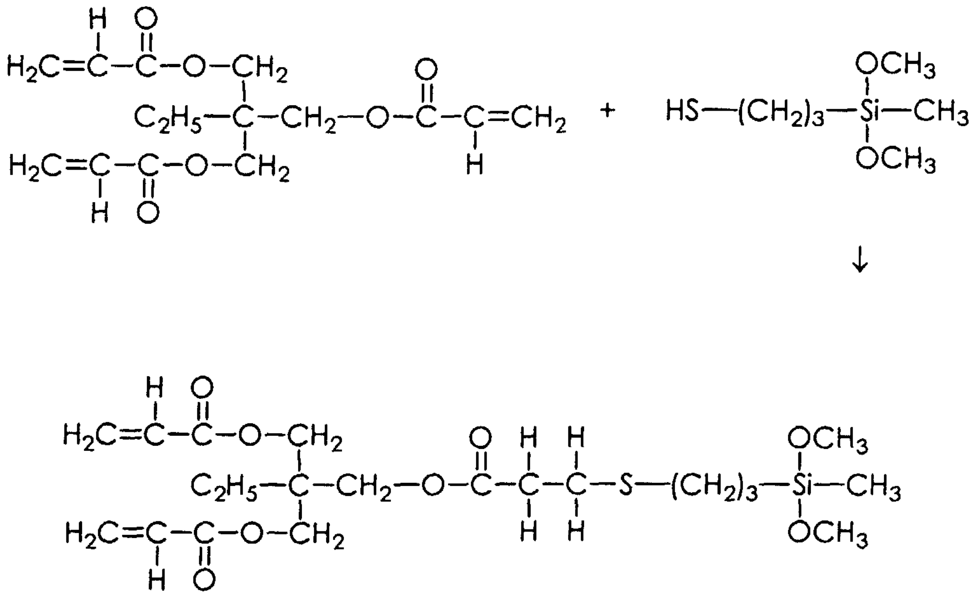

Für die Weiterverarbeitung des resultierenden Silans zu den erfindungsgemäßen Membranen gibt es zwei Varianten:

- Das resultierende Silan wird zunächst zum Aufbau des anorganischen Netzwerkes hydrolytisch polykondensiert, und das Polykondensat wird dann mit dem Polydimethylsiloxan über die Addition von Acrylat- und Amino-Gruppen verknüpft.
- Das resultierende Silan wird zunächst mit dem Polydimethylsiloxan über die Addition von Acrylat- und Amino-Gruppen verknüpft, und das Additionsprodukt wird dann zum Aufbau des anorganischen Netzwerkes hydrolytisch polykondensiert.

Die aus dem resultierenden Polykondensat gefertigte Membran wird dann zum Aufbau des organischen Netzwerkes durch Polymerisation der Acrylat-Gruppen gehärtet, und das Lösungsmittel wird quantitativ entfernt.

Beim folgenden Ausführungsbeispiel werden Dimethylsiloxan-Einheiten durch Co-Polymerisation mit einem relativ kurzkettigen Polydimethylsiloxan, das terminale Acrylat-Gruppen enthält und aus ca. 14 Dimethylsiloxan-Einheiten besteht (PDMS U22 von ABCR), in das Polykondensat eingearbeitet. Das Ergebnisse dazu enthält folgende Tabelle.

| Harztyp | Zahl d. $-Si(CH_3)_2-O$-Gruppen/Basiseinheit | $O_2$-Permeabilität [ x ] |
|---|---|---|
| 22 | 1.5 | $6.8 \cdot 10^{-10}$ |

$O_2$-Permeabilität : $x = cm^3/cm \cdot s \cdot cmHg$

| | Edukte | molares Verhältnis |
|---|---|---|
| Harztyp 22: | Glycerin-1,3-dimethacrylat | 8.0 |
| | 3-Isocyanatopropyltriethoxysilan | 8.0 |

(fortgesetzt)

| | Edukte | molares Verhältnis |
|---|---|---|
| | 1,12-Dodecandioldimethacrylat<br>PDMS U22 | 1.6<br>1.0 |

Zunächst werden, in Analogie zum Harztyp 1, Glycerin-1,3-dimethacrylat und 3-Isocyanatopropyltriethoxysilan miteinander verknüpft. Das resultierende Silan wird zum Aufbau des anorganischen Netzwerkes hydrolytisch polykondensiert, wobei das 1,12-Dodecandioldimethacrylat und/oder das PDMS U22 vor, während oder nach der Polykondensation zugegeben werden kann. Dann wird durch Co-Polymerisation der Acrylat- und der Methacrylat-Gruppen das PDMS U22 im Polykondensat chemisch verankert. Aus der resultierenden Mischung werden nach üblichen Verfahren Membranen gefertigt, bei deren Härtung durch Polymerisation der restlichen Methacrylat-Gruppen das organische Netzwerk weiter aufgebaut wird.

In der folgenden Tabelle sind die $O_2$-Permeabilitäten silanisierter und nicht-silanisierter Systeme zusammengestellt. Es ist deutlich zu erkennen, daß die Silanisierung freier -OH-Einheiten durch z.B. Trimethylsilyl-Einheiten eine Erhöhung der $O_2$-Permeabilität zur Folge hat.

| Harztyp | $O_2$-Permeabilität [ x ] silanisiert | $O_2$-Permeabilität [ x ] nicht-silanisiert |
|---|---|---|
| 2 | $2.3\ 10^{-10}$ | $0.23 \cdot 10^{-10}$ |
| 4 | $25\ 10^{-10}$ | $11.5 \cdot 10^{-10}$ |
| 7 | $13.9\ 10^{-10}$ | $3.2 \cdot 10^{-10}$ |

$O_2$-Permeabilität : $x = cm^3/cm \cdot s \cdot cmHg$

Edukte und Herstellung der Harztypen 2, 4 und 7 sind bei der Variation der anorganischen und der organischen Strukturdichte beschrieben. Die Silanisierung der Polykondensate erfolgt gemäß folgendem Reaktionsschema durch Umsetzung mit Trimethylchlorsilan.

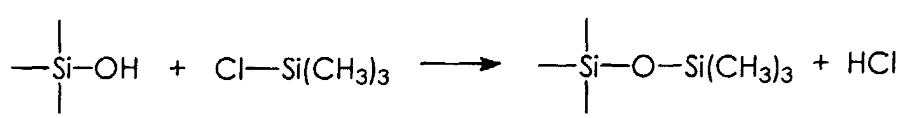

Alternativ kann die Silanisierung durch Zugabe von Hexamethyldisilazan (HMDS) dem mit Tetrahydrofuran (THF) verdünnten Ansatz unter Argon-Atmosphäre durchgeführt werden. Dadurch werden ebenfalls freie Si-OH-Gruppen zu Si-$CH_3$-Gruppen umgesetzt.

Die daraus gefertigten Membranen sind durch Polymerisation der Acrylat- bzw. Methacrylat-Gruppen gehärtet. Auf die Prozessparameter beim Spinnen bzw. Foliengießen hat die Silanisierung keine Auswirkung, sofern sie unmittelbar vor der Weiterverarbeitung erfolgt und damit den Alterungseffekt nicht beeinflusst.

Ausführungsbeispiel für zweiphasige Systeme

| Harztyp | $O_2$-Permeabilität [ x ] |
|---|---|
| 23 | $21 \cdot 10^{-10}$ |

$O_2$-Permeabilität : $x = cm^3/cm \cdot s \cdot cmHg$

| | Edukte | molares Verhältnis |
|---|---|---|
| Harztyp 23: | Trimethylolpropantriacrylat | 1 |
| | Mercaptopropylmethyldimethoxysilan | 1 |
| | Dimethyldiethoxysilan | 4 |
| | DMS-A 21 | 0.33 |

Beim Harztyp 23 wird zunächst, wie bei Harztyp 4 beschrieben, das Trimethylolpropantriacrylat und das Mercaptopropylmethyldimethoxysilan miteinander verknüpft. Anschließend wird das resultierende Silan, zusammen mit dem Dimethyldiethoxysilan, hydrolytisch polykondensiert. Das resultierende Polykondensat ist mit dem Amino terminierten Polydimethylsiloxan nicht mischbar, man erhält ein zweiphasiges Mischsystem, das sich zu milchig-trüben Membranen verarbeiten lässt, deren Härtung durch Polymerisation der Acrylat-Gruppen erfolgt.

Die erfindungsgemäßen Membranen können bei der extrakorporalen Beatmung eingesetzt werden. Die erfindungsgemäßen Membranen zeigen eine ausgezeichnete Permeationsrate für $O_2$ und $CO_2$, so dass sie für den Einsatz in Oxygenatoren hervorragend geeignet sind.

Im Folgenden wird die Herstellung der erfindungsgemäßen Membranen an konkreten Ausführungsbeispielen näher erläutert.

Hohlfaserherstellung:

Das mit einem Photoinitiator (z.B. 2 % Irgacure 184, Fa. Ciba Geigy) versetzte Harz (Viskosität bei Verarbeitungstemperatur (10 °C) ca. 100 Pas) wird durch eine ringförmige Düse extrudiert (Außendurchmesser ca. 1 mm, Ringstärke ca. 0.2 mm). Durch eine $N_2$-gespülte zweite co-zentrische Innendüse wird die Hohlfaden-Geometrie zunächst stabilisiert, bis durch eine Kombination zweier UV-Strahlungseinheiten (z.B. Blue-Point II, Fa. Hönle mit einem Rundstrahler F 300, Firma Fusion) die organische Aushärtung erfolgt. Ohne Einschränkung der Allgemeinheit können folgende Spinnparameter gewählt werden.

| | | |
|---|---|---|
| Spinnparameter | Spinntemperatur | 8 °C |
| | Spinndruck | 15 bar |
| | Abzugsgeschwindigkeit | 0,8 m/s |

Anschließend wird die Hohlfaser aufgespult. Durch Variation der Spinnparameter (Spinnmassentemperatur, Druck, Abzugsgeschwindigkeit, Gas-Durchflussrate durch den Innenkanal) lässt sich die Fasergeometrie in weiten Bereichen variieren.

Folienherstellung :

Das mit einem Photoinitiator (z.B. 1 % Irgacure 184, Fa. Ciba Geigy) versetzte Harz wird mittels eines Spaltrakels auf eine hoch polierte Walze aufgebracht. Nach dem Durchlauf durch eine UV-Härtungseinheit (z. B. UVAPRINT CM, Firma Hönle) wird die Folie abgelöst und aufgewickelt. Durch Variation der Walzenumlaufgeschwindigkeit, der -temperatur und der Spalthöhe wird die Folienstärke eingestellt.

**Beispiel 1**

| | | |
|---|---|---|
| Edukte : | Trimethylolpropantriacrylat (TMPTA) | 1 mol |
| | Mercaptopropylmethyldimethoxysilan | 1 mol |
| | Dimethyldiethoxysilan | 4 mol |
| $O_2$-Permeabilität | [$10^{-10}$ $cm^3$ (STP) / cm·s cm Hg] | 11.5 |

Synthese des Harzes :

Zur Vorlage von 89.03 g (0.3 mol) Trimethylpropantriacrylat (TMPTA) in 300 ml Essigester werden unter Argon-Atmosphäre und Eiskühlung 54.15 g (0.3 mol) Mercaptopropylmethyldimethoxysilan zugegeben. Anschließend werden langsam 19.55 g einer ethanolische KOH-Lösung zugetropft. Nach Zugabe von 8.73 g 0.5n HCl und 10 min Rühren werden 178.03 g (1.2 mol) Dimethyldiethoxysilan und 35.0 g 0.12n HCl zugegeben. Nach 23 h Rühren bei RT wird der Ansatz mit Wasser ausgeschüttelt, filtriert und auf einen Feststoffgehalt von 89 % einrotiert.

**Beispiel 2**

Einbau des die $O_2$-Permeation begünstigenden Crosslinker 1.3-Bis(3-Methacryloxypropyl)-tetrakis-(trimethylsiloxy)disiloxan (TETRAKIS)

| | | |
|---|---|---|
| Edukte : | Dodecandiodimethacrylat | 1 mol |
| | Mercaptopropylmethyldimethoxysilan | 1 mol |
| | TETRAKIS | $^1/_3$ mol |
| $O_2$-Permeabilität | [$10^{-10}$ $cm^3$ (STP) / cm·s cm Hg] : | 20 |

**Beispiel 3**

Einbau von Dimethylsiloxan-Strukturen durch Kondensation

| | | | |
|---|---|---|---|
| Harz A | Edukte : | Glycerin-1.3-dimethacrylat | 1 mol |
| | | Isocyanatopropyltriethoxysilan | 1 mol |
| | | Dimethyldiethoxysilan | 4 mol |
| | $O_2$-Permeabilität | [$10^{-10}$ $cm^3$ (STP) / cm·s cm Hg] : | 22 |
| Harz B | Edukte : | Glycerin-1.3-dimethacrylat | 1 mol |
| | | Isocyanatopropyltriethoxysilan | 1 mol |
| | | Dimethyldiethoxysilan | 6 mol |
| | $O_2$-Permeabilität | [$10^{-10}$ $cm^3$ (STP) / cm·s cm Hg] : | 66 |
| Harz C | Edukte : | Glycerin-1.3-dimethacrylat | 1 mol |
| | | Isocyanatopropyltriethoxysilan | 1 mol |
| | | Dimethyldiethoxysilan | 8 mol |
| | $O_2$-Permeabilität | [$10^{-10}$ $cm^3$ (STP) / cm·s cm Hg] : | 110 |
| Harz D | Edukte : | Glycerin-1.3-dimethacrylat | 1 mol |
| | | Isocyanatopropyltriethoxysilan | 1 mol |
| | | Dimethyldiethoxysilan | 10 mol |
| | $O_2$-Permeabilität | [$10^{-10}$ $cm^3$ (STP) / cm·s cm Hg] : | 140 |

Synthese von Harz B :

Zur Vorlage von 66.4 g (0.29 mol Glycerin-1.3-dimethacrylat und Dibutylzinndilaurat (als Additionskatalysator) werden unter Kühlung 72.0 g (0.29 mol) 3-Isocyanatopropyltriethoxysilan zugetropft. Nach 21 h Rühren werden 290 ml Essigester, 258.9 g (1.75 mol) Dimethyldiethoxysilan und 63. 3 g Wasser (incl. Katalysator) zugesetzt. Nach 6 d Rühren wird mit Wasser ausgeschüttelt, filtriert, einrotiert und die flüchtigen Bestandteile an der Ölpumpe vollständig

entfernt. Feststoffgehalt: 95.6 %, Viskosität nach 1h: 2.2 Pas (25 °C).
Die Synthese der Harz-Varianten A, C und D erfolgt ganz analog.

**Beispiel 4**

Einbau von Dimethylsiloxan-Strukturen durch Addition über Amino-Endgruppen

Amino-terminiertes Polydimethylsiloxan DMS A 21 (Gelest) wird durch Reaktion der Amino-Gruppen mit den Acrylat-Gruppen des Trimethylolpropantriacrylat (TMPTA) addiert.

| | | | |
|---|---|---|---|
| Harz A | Edukte : | TMPTA | 1 mol |
| | | Mercaptopropylmethyldimethoxysilan | 1 mol |
| | | DMS A 21 | 0.03 mol |
| | $O_2$-Permeabilität | [$10^{-10}$ $cm^3$ (STP) / cm·s cm Hg] : | 19 |
| Harz B | Edukte : | TMPTA | 1 mol |
| | | Mercaptopropylmethyldimethoxysilan | 1 mol |
| | | DMS A 21 | 0.09 mol |
| | $O_2$-Permeabilität | [$10^{-10}$ $cm^3$ (STP) / cm·s cm Hg] : | 160 |
| | E-Modul [MPa] | | 210 |
| | Biegefestigkeit [MPa] | | 25 |

Das Lösungsmittel n-Butylacetat wurde nach Aushärtung quantitativ entfernt.

**Beispiel 5**

Einbau von Dimethylsiloxan-Strukturen durch Co-Polymerisation von Acryloxy terminiertem Polydimethylsiloxan PDMS U22 (ABCR)

Das relativ kurzkettige, ca. 14 Polydimethylsiloxan-Einheiten enthaltende PDMS U22 der Firma ABCR ist in jedem Mischungsverhältnis mischbar.

| | | |
|---|---|---|
| Edukte : | Glycerin-1.3-dimethacrylat | 8 mol |
| | 3-Isocyanatopropyltriethoxysilan | 8 mol |
| | 1.12-Dodecandioldimethacrylat | 1.6 mol |
| | Acryloxy-terminiertes Polydimethylsiloxan PDMS U22 | 1 mol |

entsprechend 1.5 DMS-Einheiten pro Basiseinheit.
In allen Fällen konnte die Spinnbarkeit des Harzes nachgewiesen werden.

**Patentansprüche**

**1.** Oxygenatormembran, dadurch erhältlich, dass man eine gering viskose bis harzartige Flüssigkeit nach üblichen Methoden zu einer Membran verarbeitet, dass man die Membran gegebenenfalls trocknet und anschließend thermisch und/oder strahlungs-induziert und/oder chemisch induziert härtet, wobei die Flüssigkeit erhalten worden ist
a) durch hydrolytische Polykondensation

- einer oder mehrerer Verbindungen der allgemeinen Formel I,

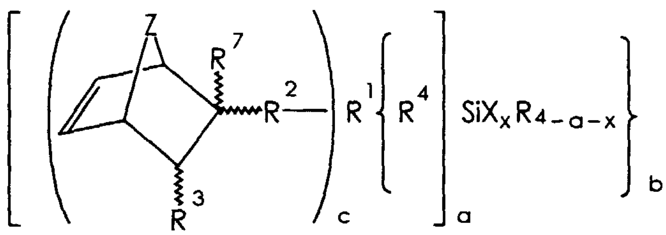

(I)

in der die Reste und Indices folgende Bedeutung haben:

- R = Alkyl, Alkenyl, Aryl, Alkylaryl oder Arylalkyl mit jeweils 1 bis 15 Kohlenstoff-Atomen, wobei diese Reste Sauerstoff- und/oder Schwefel-Atome und/oder Ester- und/oder Carbonyl- und/oder Carboxyl- und/oder Amid- und/oder Amino-Gruppen enthalten können,
- $R^1$ = Alkylen, Arylen, Arylenalkylen oder Arylenalkylen mit jeweils 0 bis 15 Kohlenstoff-Atomen, wobei diese Reste Sauerstoff- und/oder Schwefel-Atome und/oder Ester- und/oder Carbonyl- und/oder Carboxyl-und/ oder Amid- und/oder Amino-Gruppen enthalten können,
- $R^2$ = Alkylen, Arylen, Arylenalkylen oder Arylenalkylen mit jeweils 0 bis 15 Kohlenstoff-Atomen, wobei diese Reste Sauerstoff- und/oder Schwefelatome und/oder Ester- und/oder Carbonyl- und/oder Carboxyl- und/ oder Amid- und/oder Amino-Gruppen enthalten können,
- $R^3$ = Wasserstoff, $R^2$-$R^1$-$R^4$-$SiX_xR_{3-x}$, Carboxyl-, Alkyl, Alkenyl, Aryl, Alkylaryl oder Arylalkyl mit jeweils 1 bis 15 Kohlenstoff-Atomen, wobei diese Reste Sauerstoff- und/oder Schwefel-Atome und/oder Ester- und/ oder Carbonyl- und/oder Carboxyl- und/oder Amid- und/oder Amino-Gruppen enthalten können,
- $R^4$ = $-(CHR^6-CHR^6)_n-$, mit n = 0 oder 1, $-CHR^6-CHR^6-S-R^5-$, $-CO-S-R^5-$, $-CHR^6-CHR^6-NR^6-R^5-$, $-Y-CS-NH-R^5-$, $-S-R^5-$,$-Y-CO-NH-R^5-$, $-CO-O-R^5-$, $-Y-CO-C_2H_3(COOH)-R^5-$, $-Y-CO-C_2H_3(OH)-R^5-$ oder $-CO-NR^6-R^5-$,
- $R^5$ = Alkylen, Arylen, Arylenalkylen oder Arylenalkylen mit jeweils 1 bis 15 Kohlenstoff-Atomen, wobei diese Reste Sauerstoff- und/oder Schwefel-Atome und/oder Ester- und/oder Carbonyl- und/oder Carboxyl-. und/ oder Amid- und/oder Amino-Gruppen enthalten können,
- $R^6$ = Wasserstoff, Alkyl oder Aryl mit 1 bis 10 Kohlenstoff-Atomen,
- $R^7$ = Wasserstoff, Alkyl, Alkenyl, Aryl, Alkylaryl oder Arylalkyl mit jeweils 1 bis 15 Kohlenstoff-Atomen, wobei diese Reste Sauerstoff- und/oder Schwefelatome und/oder Ester- und/oder Carbonyl- und/oder Carboxyl- und/oder Amid- und/oder Amino-Gruppen enthalten können,
- X = Wasserstoff, Halogen, Hydroxy, Alkoxy, Acyloxy, Alkylcarbonyl, Alkoxycarbonyl oder $NR''_2$, mit R" = Wasserstoff, Alkyl oder Aryl,
- Y = -O-, -S- oder $-NR^6-$,
- Z = -O- oder $-(CHR^6)_m-$, mit m = 1 oder 2,
- a = 1, 2 oder 3, mit b = 1 für a = 2 oder 3,
- b = 1, 2 oder 3, mit a = 1 für b = 2 oder 3,
- c = 1 bis 6,
- x = 1, 2 oder 3, mit a+x = 2, 3 oder 4,

und/oder

- einer oder mehrerer Verbindungen der allgemeinen Formel II,

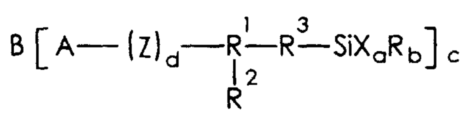

(II)

in der die Reste und Indices folgende Bedeutung haben:

- B = ein geradkettiger oder verzweigter organischer Rest mit mindestens einer C=C-Doppelbindung und 4 bis 50 Kohlenstoff-Atomen,
- R = Alkyl, Alkenyl, Aryl, Alkylaryl oder Arylalkyl mit jeweils 1 bis 15 Kohlenstoff-Atomen, wobei diese Reste Sauerstoff- und/oder Schwefel-Atome und/oder Ester- und/oder Carbonyl- und/oder Carboxyl- und/oder

Amid- und/oder Amino-Gruppen enthalten können,

$R^3$ = Alkylen, Arylen, Arylenalkylen oder Alkylenarylen mit jeweils 0 bis 10 Kohlenstoff-Atomen, wobei diese Reste durch Sauerstoff- und/oder durch Schwefel-Atome und/oder durch Amino-Gruppen unterbrochen sein können,

X = Wasserstoff, Halogen, Hydroxy, Alkoxy, Acyloxy, Alkylcarbonyl, Alkoxycarbonyl oder $NR''_2$, mit R''= Wasserstoff, Alkyl, Aryl oder Alkylaryl,

A = O, S oder NH

für d = 1 und Z = CO und

$R^1$ = Alkylen, Arylen oder Alkylenarylen mit jeweils 1 bis 10 Kohlenstoff-Atomen, wobei diese Reste durch Sauerstoff- und/oder durch Schwefel-Atome und/oder durch Amino-Gruppen unterbrochen sein können, und

$R^2$ = COOH oder H,

oder

A = O, S, NH oder COO

für d = 1 und Z = CHR', mit R' = H, Alkyl, Aryl oder Alkylaryl, und

$R^1$ = Alkylen, Arylen oder Alkylenarylen mit jeweils 1 bis 10 Kohlenstoff-Atomen, wobei diese Reste durch Sauerstoff- und/oder durch Schwefel-Atome und/oder durch Amino-Gruppen unterbrochen sein können, und

$R^2$ = OH,

oder

A = O, S, NH oder COO

für d = 0 und

$R^1$ = Alkylen, Arylen oder Alkylenarylen mit jeweils 1 bis 10 Kohlenstoff-Atomen, wobei diese Reste durch Sauerstoff- und/oder durch Schwefel-Atome und/oder durch Amino-Gruppen unterbrochen sein können, und

$R^2$ = OH,

oder

A = S

für d = 1 und Z = CO und

$R^1$ = N und

$R^2$ = H,

a = 1, 2 oder 3,

b = 0, 1 oder 2, mit a+b = 3,

c = 1, 2, 3 oder 4,

und/oder

■ einer oder mehrerer Verbindungen der allgemeinen Formel III,

$$\{X_aR_bSi[(R'A)_c]_{(4-a-b)}\}_xB \qquad \text{(III)}$$

in der die Reste und Indices folgende Bedeutung haben:

A = O, S, PR'', POR'', NHC(O)O oder NHC(O)NR'',

B = geradkettiger oder verzweigter organischer Rest, der sich von einer Verbindung B' mit mindestens einer (für c = 1 und A = NHC(O)O oder NHC(O)NR'') bzw. mindestens zwei C=C-Doppelbindungen und 5 bis 50 Kohlenstoff-Atomen ableitet,

R = Alkyl, Alkenyl, Aryl, Alkylaryl oder Arylalkyl mit jeweils 1 bis 15 Kohlenstoff-Atomen, wobei diese Reste

Sauerstoff- und/oder Schwefel-Atome, und/oder Ester- und/oder Carbonyl- und/oder Carboxyl- und/oder Amid- und/oder Amino-Gruppen enthalten können,

R' = Alkylen, Arylen oder Alkylenarylen,

R" = Wasserstoff, Alkyl, Aryl oder Alkylaryl,

X = Wasserstoff, Halogen, Hydroxy, Alkoxy, Acyloxy, Alkylcarbonyl, Alkoxycarbonyl oder $NR''_2$, mit R"= Wasserstoff, Alkyl, Aryl oder Alkylaryl,

a = 1,2oder3,

b = 0, 1 oder 2,

c = 0 oder 1,

x = eine ganze Zahl, deren Maximalwert der Anzahl von Doppelbindungen in der Verbindung B' minus 1 entspricht, bzw. gleich der Anzahl von Doppelbindungen in der Verbindung B' ist, wenn c = 1 und A für NHC(O)O oder NHC(O)NR" steht,

wobei die obigen Alkyl- bzw. Alkenyl-Reste substituierte oder unsubstituierte geradkettige, verzweigte oder cyclische Reste mit 1 bzw. 2 bis 20 Kohlenstoff-Atomen sind, Aryl für substituiertes oder unsubstituiertes Phenyl, Naphthyl oder Biphenyl steht und sich die obigen Alkoxy-, Acyloxy-, Alkylcarbonyl-, Alkoxycarbonyl-, Alkylaryl-, Arylalkyl-, Arylen-, Alkylen- und Alkylenaryl-Reste von den oben definierten Alkyl- und Aryl-Resten ableiten, und/oder

■ einer oder mehrerer Verbindungen der allgemeinen Formel IV,

$$Y_aSiX_xR_{4-a-x} \qquad (IV)$$

in der die Reste und Indices folgende Bedeutung haben:

R = Alkyl, Alkenyl, Aryl, Alkylaryl oder Arylalkyl mit jeweils 1 bis 15 Kohlenstoff-Atomen, wobei diese Reste Sauerstoff- und/oder Schwefel-Atome und/oder Ester- und/oder Carbonyl- und/oder Carboxyl- und/oder Amid- und/oder Amino-Gruppen enthalten können,

X = Wasserstoff, Halogen, Hydroxy, Alkoxy, Acyloxy, Alkylcarbonyl, Alkoxycarbonyl oder $NR''_2$, mit R" = Wasserstoff, Alkyl, Aryl oder Alkylaryl,

Y = organischer Rest mit 1 bis 30, bevorzugt mit 1 bis 20 Kohlenstoff-Atomen, und mit 1 bis 5, bevorzugt mit 1 bis 4 Mercapto-Gruppen,

a = 1, 2 oder 3,

x = 1, 2 oder 3, mit a+x = 2, 3 oder 4,

und/oder

■ von den Verbindungen der Formeln I bis IV abgeleiteten Vorkondensaten und gegebenenfalls

■ einer oder mehrerer Verbindungen der allgemeinen Formel V,

$$X_aSiR_{4-a} \qquad (V)$$

in der die Reste und Indices folgende Bedeutung haben:

R = Alkyl, Alkenyl, Aryl, Alkylaryl oder Arylalkyl mit jeweils 1 bis 15 Kohlenstoff-Atomen, wobei diese Reste Sauerstoff- und/oder Schwefel-Atome und/oder Ester- und/oder Carbonyl- und/oder Carboxyl- und/ oder Amid- und/oder Amino-Gruppen enthalten können,

X = Wasserstoff, Halogen, Hydroxy, Alkoxy, Acyloxy, Alkylcarbonyl, Alkoxycarbonyl oder $NR''_2$, mit R" = Wasserstoff, Alkyl, Aryl oder Alkylaryl,

a = 1, 2 oder 3,

und/oder von diesen abgeleiteten Vorkondensaten,
wobei die hydrolytische Polykondensation durch Zugabe von Wasser oder Feuchtigkeit und in Anwesenheit oder

in Abwesenheit eines Lösungsmittels und/ oder eines Kondensationskatalysators durchgeführt wird, und wobei, bezogen auf die Monomeren, das molare Verhältnis der Summe der Verbindungen der Formeln I, II, III und IV zu Verbindungen der Formel V zwischen 1:0 und 1: 20 liegt,
und gegebenenfalls b) durch Zugabe

- von einem oder mehreren copolymerisierbaren und/oder (poly)addierbaren Monomeren und/oder Oligomeren,
- und/oder von einem oder mehreren Härtungskatalysatoren.

**2.** Oxygenatormembran nach Anspruch 1, dadurch erhältlich, dass man die Flüssigkeit zu Flachmembranen oder zu rohrförmigen Membranen verarbeitet.

**3.** Oxygenatormembran nach Anspruch 1 oder 2, dadurch gekennzeichnet, dass sie sich auf einem Träger befindet.

**4.** Oxygenatormembran nach einem oder mehreren der Ansprüche 1 bis 3, dadurch gekennzeichnet, dass sie aus einer Flüssigkeit erhalten worden ist, die Polykondensate enthält, die sich von einer oder mehreren Verbindungen der Formel I ableiten, in der die Indices a und/oder b und/oder c den Wert = 1 haben.

**5.** Oxygenatormembran nach einem oder mehreren der Ansprüche 1 bis 4, dadurch gekennzeichnet, dass sie aus einer Flüssigkeit erhalten worden ist, die Polykondensate enthält, die sich von einer oder mehreren Verbindungen der Formeln II und/oder III ableiten, in denen der Rest B eine oder mehrere Acrylat- und/ oder Methacrylat-Gruppen aufweist.

**6.** Oxygenatormembran nach einem oder mehreren der Ansprüche 1 bis 5, dadurch gekennzeichnet, dass sie aus einer Flüssigkeit erhalten worden ist, die Polykondensate enthält, die sich von einer oder mehreren Verbindungen der Formel IV' ableiten,

$$[(HS\text{-}R^5)_nR^6\text{-}S\text{-}E\text{-}R^5]_aSiX_xR_{4-a-x} \qquad (IV')$$

in der die Reste und Indices folgende Bedeutung haben:

E = -CO-NH-, -CS-NH-, $-CH_2-CH_2-$ oder $-CH_2-CH(OH)-$
R = wie in Anspruch 1 definiert;
$R^5$ = Alkylen, Arylen, Arylenalkylen oder Arylenalkylen mit jeweils 1 bis 15 Kohlenstoff-Atomen, wobei diese Reste durch Sauerstoff- und/oder durch Schwefel-Atome und/oder durch Ester- und/oder durch Carbonyl- und/oder durch Carboxyl- und/oder durch Amid- und/oder durch Amino-Gruppen unterbrochen sein können;
$R^6$ = Alkylen, Arylen, Arylenalkylen oder Arylenalkylen mit jeweils 1 bis 15 Kohlenstoff-Atomen, wobei diese Reste durch Sauerstoff- und/oder durch Schwefel-Atome und/oder durch Ester- und/oder durch Carbonyl- und/oder durch Carboxyl- und/oder durch Amid- und/oder durch Amino-Gruppen unterbrochen sein können;
X = wie in Anspruch 1 definiert;
a = wie in Anspruch 1 definiert;
n = 2, 3, 4 oder 5;
x = wie in Anspruch 1 definiert;

**7.** Oxygenatormembran nach einem oder mehreren der Ansprüche 1 bis 6, dadurch gekennzeichnet, dass sie aus einer Flüssigkeit erhalten worden ist, die eine oder mehrere organische Verbindungen mit einer oder mehreren Mercapto-Gruppen enthält.

**8.** Oxygenatormembran nach einem oder mehreren der Ansprüche 1 bis 7, dadurch gekennzeichnet, dass sie aus einer Flüssigkeit erhalten worden ist, die eine oder mehrere organische Verbindungen mit einer oder mehreren C=C-Doppelbindungen enthält.

**9.** Oxygenatormembran nach Anspruch 8, dadurch gekennzeichnet, dass sie aus einer Flüssigkeit erhalten worden ist, die Polykondensate und/oder Oligokondensate enthält, die eine oder mehrere C=C-Doppelbindungen aufweisen und die sich durch Kondensation von organisch modifizierten, hydrolytisch kondensierbaren Silanen ableiten.

**10.** Oxygenatormembran nach einem oder mehreren der Ansprüche 1 bis 9, dadurch gekennzeichnet, dass sie aus einer Flüssigkeit erhalten worden ist, die eine oder mehrere organische Verbindungen mit einer oder mehreren substituierten und/oder unsubstituierten Amino-Gruppen enthält.

**11.** Oxygenatormembran nach Anspruch 10, dadurch gekennzeichnet, dass sie aus einer Flüssigkeit erhalten worden ist, die Polykondensate und/oder Oligokondensate enthält, die eine oder mehrere substituierte und/oder unsubstituierte Amino-Gruppen aufweisen und die sich durch Kondensation von organisch modifizierten, hydrolytisch kondensierbaren Silanen ableiten.

**12.** Verfahren zur Herstellung von Oxygenatormembranen nach einem oder mehreren der Ansprüche 1 bis 11, dadurch gekennzeichnet, dass man

a) durch hydrolytische Kondensation

- einer oder mehrerer Verbindungen der allgemeinen Formel I

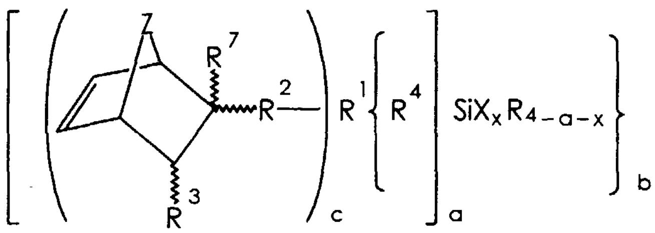

(I)

in der die Reste und Indices wie in Anspruch 1 definiert sind, und/oder

- einer oder mehrerer Verbindungen der allgemeinen Formel II

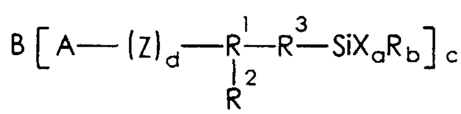

(II)

in der die Reste und Indices wie in Anspruch 1 definiert sind, und/oder

- einer oder mehrerer Verbindungen der allgemeinen Formel III

$$\{X_aR_bSi[(R'A)_c]_{(4-a-b)}\}_xB \qquad (III)$$

in der die Reste und Indices wie in Anspruch 1 definiert sind, und/oder

- einer oder mehrerer Verbindungen der allgemeinen Formel IV,

$$Y_aSiX_xR_{4-a-x} \qquad (IV)$$

in der die Reste und Indices wie in Anspruch 1 definiert sind, und/oder

- von Verbindungen der Formel I bis IV abgeleiteten Vorkondensaten und gegebenenfalls

- einer oder mehrerer Verbindungen der allgemeinen Formel V,

$$X_aSiR_{4-a} \quad (V)$$

in der die Reste und Indices wie in Anspruch 1 definiert sind,

durch Zugabe von Wasser oder Feuchtigkeit und in Anwesenheit oder in Abwesenheit eines Lösungsmittels und/oder eines Kondensationskatalysators und wobei, bezogen auf die Monomeren, das molare Verhältnis der Summe der Verbindungen der Formeln I, II und III zu Verbindungen der Formel IV zwischen 1:0 und 1: 20 liegt,
und gegebenenfalls

b) durch Zugabe

- von einem oder mehreren copolymerisierbaren und/oder (poly)addierbaren Monomeren und/oder Oligomeren,
- und/oder von einem oder mehreren Härtungskatalysatoren

eine gering viskose bis harzartige Flüssigkeit fertigt, dass man diese nach üblichen Methoden zu einer Membran verarbeitet, dass man diese Membran gegebenenfalls trocknet und anschließend thermisch und/oder strahlungsinduziert und/oder chemisch induziert härtet.

**13.** Verfahren nach Anspruch 12, dadurch gekennzeichnet, dass man die Flüssigkeit zu Flachmembranen oder zu rohrförmigen Membranen verarbeitet.

**14.** Verfahren nach Anspruch 12 oder 13, dadurch gekennzeichnet, dass man die Membran auf einen Träger fertigt.

**15.** Verfahren nach einem oder mehreren der Ansprüche 12 bis 14, dadurch gekennzeichnet, dass man eine Flüssigkeit verwendet, die Polykondensate enthält, die sich von einer oder mehreren Verbindungen der Formel I ableiten, in der die Indices a und/oder b und/oder c den Wert = 1 haben.

**16.** Verfahren nach einem oder mehreren der Ansprüche 12 bis 15, dadurch gekennzeichnet, dass man eine Flüssigkeit verwendet, die Polykondensate enthält, die sich von einer oder mehreren Verbindungen der Formeln II und/ oder III ableiten, in denen der Rest B eine oder mehrere Acrylat- und/oder Methacrylat-Gruppen enthält.

**17.** Verfahren nach einem oder mehreren der Ansprüche 12 bis 16, dadurch gekennzeichnet, dass man eine Flüssigkeit verwendet, die Polykondensate enthält, die sich von einer oder mehreren Verbindungen der Formel IV' ableiten,

$$[(HS\text{-}R^5)_nR^6\text{-}S\text{-}E\text{-}R^5]_aSiX_xR_{4-a-x} \quad (IV')$$

in der die Reste und Indices wie in Anspruch 6 definiert sind.

**18.** Verfahren nach einem oder mehreren der Ansprüche 12 bis 17, dadurch gekennzeichnet, dass man eine Flüssigkeit verwendet, die eine oder mehrere organische Verbindungen mit einer oder mehreren Mercapto-Gruppen enthält.

**19.** Verfahren nach einem oder mehreren der Ansprüche 12 bis 18, dadurch gekennzeichnet, dass man eine Flüssigkeit verwendet, die eine oder mehrere organische Verbindungen mit einer oder mehreren C=C-Doppelbindungen enthält.

**20.** Verfahren nach Anspruch 19, dadurch gekennzeichnet, dass man eine Flüssigkeit verwendet, die Polykondensate und/oder Oligokondensate enthält, die eine oder mehrere C=C-Doppelbindungen aufweisen und die sich durch Kondensation von organisch modifizierten, hydrolytisch kondensierbaren Silanen ableiten.

**21.** Verfahren nach einem oder mehreren der Ansprüche 12 bis 20, dadurch gekennzeichnet, dass man eine Flüssigkeit verwendet, die eine oder mehrere organische Verbindungen mit einer oder mehreren substituierten und/oder

unsubstituierten Amino-Gruppen enthält.

**22.** Verfahren nach Anspruch 21, dadurch gekennzeichnet, dass man eine Flüssigkeit verwendet, die Polykondensate und/oder Oligokondensate enthält, die eine oder mehrere substituierte und/oder unsubstituierte Amino-Gruppen aufweisen und die sich durch Kondensation von organisch modifizierten, hydrolytisch kondensierbaren Silanen ableiten.